# EUROPEAN PATENT APPLICATION

(11) **EP 4 223 776 A1**
(43) Date of publication of application: **09.08.2023**
(21) Application number: 21874435.7
(22) Date of filing: 27.09.2021
(51) Int. Cl.: C07K 16/28, C07K 16/46, C12N 15/13, C12N 15/62, C12N 15/63, A61K 39/395, A61P 31/06, A61P 31/18, A61P 31/14, A61P 31/20, A61P 35/00, G01N 33/577

(54) **ANTI-PD-L1 ANTIBODY AND USE THEREOF**

(30) Priority: 29.09.2020 CN 202011049435
(71) Applicant: Immunourchin, Suzhou, Jiangsu 215341 (CN)
(72) Inventor: ZHOU, Jun, Suzhou, Jiangsu 215341 (CN)
(74) Representative: dompatent von Kreisler Selting Werner - Partnerschaft von Patent- und Rechtsanwälten mbB
(86) International application number: PCT/CN2021/121022
(87) International publication number: WO 2022/068775

(57) **Abstract**

Provided in the present invention are a PD-L1 antibody and a use thereof; the antibody or an active fragment thereof does not block the binding of PD-1 with PD-L1, and specifically targets the IgC-like region of PD-L1. The non-blocking PD-L1 antibody of the present invention can circumvent SPD-L1 to the maximum extent, and has the activity of inducing ADCC/CDC functional effects whilst also having excellent anti-tumour activity.

## Description

### Technical field

The present invention related to tumor immunology field and specifically referred to the antibodies binding to pan IgC segment of PD-L1 and inducing ADCC/CDC activity.

### Background

In last decade, it is a milestone breakthrough that immune check-point blockade by antibodies were applied to treat patients with different types of cancers, such as ipilimumab (CTLA4 blocking antibody), pembrolizumab/nivolumab (PD-1 blocking antibody), and atezolizumab (PD-L1 blocking antibody). It is a widely accepted concept first put forward by Dr. James Allison, an American scientist. Although different clinical indications showed distinct response rates, percentages of patient responders with the most common solid tumors are limited, around 20%.

PD1/PD-L1 pathway is one of the important immune checkpoints. PD-L1, programmed death ligand-1, is a membrane protein, which is mainly expressed on dendritic cells and monocytes. Its receptor is called PD-1, programmed death-1, which expresses on activated T and B cells, dendritic cells and monocytes. When T cells recognizing and binding to antigen held by MHC, T cells are activated, and thereafter they express PD-1. Engagement of PD-L1 with PD-1 results in inhibition of T cell function, leading to immune suppression. Expression of PD-L1 was found in different types of tumor cells.

Studies further showed that PD-1 interacts with PD-L1 by binding to PD-L1 IgV domain (amino acids 19-131). Heavy chain of atezolizumab binds to E58, Q66, V111, R113, and R125 of antigen-determining group of PD-L1 via hydrogen bond, and antigen-determining group of 154, Y63, V111, M115, S117, A121, and Y123 in CC' loop of PD-L1 bind to CDRs of the heavy chain via van der Waals. Although different PD-L1 antibodies such as atezolizumab, durvalumab, BMS-963559, and avelumab recoginize different antigen-determining groups, the regions that they all recognize are located in IgV domain of PD-L1 (amino acids 19-131), and these antibodies compete with PD-1 at the same binding region. The purposes of these antibodies are involved in blocking interaction of PD-L1 with PD-1. Until now, development of PD-L1 antibodies is based on immune check-point blockade.

Therapeutic response of immune check-point blockade is dependent of tumor features in some way, such as tumor specific immune response, tumor microenvironment, and sensitivity of tumor to immune response. Studies found soluble PD-L1 (sPD-L1) being present in patient sera. High levels of sPD-L1 that is present in aggressive renal cell carcinoma, multiple myeloma, and diffuse large B-cell lymphoma, are associated with shorter survival. Recently, study found that there are four splicing varients of PD-L1 in melanoma cells, and they resulted in secretion of sPD-L1. sPD-L1 secretion is dependent of splicing activity, cytokine induction, cell stress, cell injury and cell death in melanoma cells. Expression of membrane-bound PD-L1 is proportional to secretion of sPD-L1. Furthermore, PD-L1 splicing activities and sPD-L1 secretion are common in varieties of different tumors cells, wherein the most common or most highly expressed varients are PD-L1-3/12 and PD-L1-9. The total lengths of sPD-L1-3/12 and PD-L1-9 are amino acids 19-178 and 19-243, respectively. Obviously, all the secreted sPD-L1 variants contain IgV domain, which is the region that current PD-L1 blocking antibodies bind to.

Current studies further showed that sPD-L1 varients could function as "decoys" and bound to PD-L1 blocking antibody and induced resistance to PD-L1 blockade. sPD-L1 secreted by only 1% tumor cells can abolish anti-tumor effects of PD-L1 blocking antibody. In PD-L1 antibody therapy, sPD-L1 secretion is associated with recurrence in tumor patients. It strongly suggests that sPD-L1 secreted by tumor is a difficult barrier for PD-L1 blocking antibody to penetrate or one of resistance mechanism in treating tumor by PD-L1 blockade.

Taken together, there is urgent need in the field for developing a more effective therapeutic monoclonal antibody, which circumvents sPD-L1 to the most extent and has robust Fc-induced ADCC/CDC activity and anti-tumor effect.

### Summary of the invention

The purpose of the present invention is to provide a class of PD-L1 antibodies that recognize pan IgC segment of PD-L1 and induce ADCC/CDC functional effects, which are anti-tumor and anti-sPD-L1 secretion and drug-resistant PD-L1 antibodies.

In the first aspect, the present invention provides a PD-L1 antibody or its active fragment, which does not block the binding of PD-L1 with PD-1 and specifically binds to pan IgC segment of PD-L 1 and has ADCC/CDC functional effects activity.

In another preferred embodiment, the antibody doesn't bind to IgV domain of PD-L1 (e.g., amino acids 19-127).

In another preferred embodiment, the antibody doesn't compete with PD-1 for binding to IgV domain of PD-L1.

In another preferred embodiment, the antibody doesn't compete with PD-1 for binding to same epitopes in IgV domain of PD-L1.

In another preferred embodiment, the antibody doesn't bind to or has low affinity to soluble PD-L1 (sPD-L1).

In another preferred embodiment, the antibody doesn't bind to soluble PD-L1 PD-L1-3.

In another preferred embodiment, the binding affinity (P1) of the antibody to PD-L1-9 is much lower than the binding affinity (P0) of antibody #130021 (a control antibody) to PD-L1-9, preferably P1/P0 ≤ 1/5, more preferably ≤ 1/10, much more preferably 1/100.

In another preferred embodiment, the antibody that has low affinity to soluble PD-L1 comprises: PD-L1-1 and PD-L1-9.

In another preferred embodiment, the antibody doesn't bind to or partially binds to soluble PD-L1 (sPD-L1).

In another preferred embodiment, the soluble PD-L1 that the antibody doesn't bind to comprises: PD-L1-3.

In another preferred embodiment, the soluble PD-L1 that the antibody partially binds to comprises: PD-L1-1 and PD-L1-9.

The antibody is a non-blocking antibody and doesn't bind to any one of amino acid sites of PD-L1, which comprise:
(i) E58, Q66, V111, R113, R125; and
(ii) I54, Y56, N63, V111, M115, 5117, A121, and Y123.

In another preferred embodiment, the PD-L1 antibody or its active fragment thereof comprises:
(a) amino acid sequences as shown in SEQ ID NO: 16 of LCDR1, SEQ ID NO: 17 of LCDR2, SEQ ID NO: 18 ofLCDR3; and
(b) amino acid sequences as shown in SEQ ID NO: 12 of HCDR1, SEQ ID NO: 13 of HCDR2, SEQ ID NO: 14 of HCDR3.

Wherein, the antibody sepecifically binds to pan IgC segment of PD-L1.

In another preferred embodiment, the pan IgC segment consists of IgC domain and its connection segment to cell membrane, wherein the region consists of amino acid residues 132-138 of PD-L1 protein.

In another preferred embodiment, the antibody or its active fragment is able to induce ADCC/CDC functional effects and further kill tumor.
wherein any one of the above amino acid sequences further comprises a derivative sequence which is obtained through optional addition, deletion, modification and/or substitution of at least one amino acid and is capable of retaining PD-L1 binding affinity.

In another prefered embodiment, the amino acid sequence of any one of the above CDRs comprises a derived CDR sequence with the addition, deletion, modification and/or substitution of 1, 2 or 3 amino acids, and enables the derived antibody composed of VH and VL containing the derived CDR sequences to retain the binding affinity to PD-L1.

In another prefered embodiment, the number of added, deleted, modified and/or substituted amino acids is 1-5 (such as 1-3, preferably 1-2, more preferably 1).

In another preferred embodiment, the derivative sequences
that retain the PD-L1 binding affinity after the addition, deletion, modification and/or substitution of at least one amino acid are amino acids with homology or sequence indentity of at least 96%.

In another preferred embodiment, the PD-L1 antibody or its active fragment thereof comprises variable regions of heavy chain or variable regions of light chain, the polypeptide sequence of the variable region of heavy chain is at least 95% homologous to SEQ ID: 11, 20, 21, 22, 23, 24, or 25, the polypeptide sequence of the variable region of light chain is at least 95% homologous to SEQ ID: 15, 26, 27, 28, 29, or 30.

In another preferred embodiment, the PD-L1 antibody or its active fragment thereof comprises the polypeptides of heavy chain variable regions as shown in SEQ ID: 11, 20, 21, 22, 23, 24, or 25, and polypeptides of light chain variable region as shown in SEQ ID: 15, 26, 27, 28, 29, or 30.

In another preferred embodiment, the PD-L1 antibody or its active fragment thereof comprises heavy chain and light chain variable regions selected from the group:
(1) Heavy chain variable region as shown in SEQ ID NO: 25, and light chain variable region as shown in SEQ ID NO: 27;
(2) Heavy chain variable region as shown in SEQ ID NO: 25, and light chain variable region as shown in SEQ ID NO: 30;
(3) Heavy chain variable region as shown in SEQ ID NO: 23, and light chain variable region as shown in SEQ ID NO: 29;
(4) Heavy chain variable region as shown in SEQ ID NO: 24, and light chain variable region as shown in SEQ ID NO: 29;
(5) Heavy chain variable region as shown in SEQ ID NO: 25, and light chain variable region as shown in SEQ ID NO: 29;
(6) Heavy chain variable region as shown in SEQ ID NO: 24, and light chain variable region as shown in SEQ ID NO: 30;
(7) Heavy chain variable region as shown in SEQ ID NO: 23, and light chain variable region as shown in SEQ ID NO: 30;

In another preferred embodiment, the sequence homology or identity of the heavy chain variable region with SEQ ID: 11, 20, 21, 22, 23, 24, or 25 is at least 80%, 85%, 90%, 91%, 93%, 94%, 95%, 96%, 97%, 98%, or 99%.

In another preferred embodiment, the sequence homology or identity of the light chain variable region with SEQ ID: 15, 26, 27, 28, 29, or 30 is at least 80%, 85%, 90%, 91%, 93%, 94%, 95%, 96%, 97%, 98%, or 99%.

In another preferred embodiment, the antibody or its active fragment thereof is chimeric.

In another preferred embodiment, the antibody or its active fragment thereof is humanized.

In the second aspect of the present invention, the present invention provides a recombinant protein, the recombinat protein comprises:
(i) the antibody or its active fragment as described in the first aspect of the present invention; and
(ii) an optional tag sequence to assist expression or purification.

In another preferred embodiment, the tag sequence comprises a 6His tag.

In another preferred embodiment, the recombinant protein is specific for the anti-PD-L1 protein.

In the third aspect of the present invention, the present invention provides a polynucleotide encoding the antibody or its active fragment of the first aspect of the present invention, or the recombinant protein of the second aspect of the present invention.

In the fourth aspect, the present invention provides a vector, which comprises the polynucleotide of the third aspect of the present invention.

In another preferred embodiment, the vector comprises bacteria plasmid, phage, yeast plasmid, plant cell virus, mammalian cell virus such as adenovirus, retrovirus, or other vectors.

In the fifth aspect of the present invention, the present invention provides a genetically engineered host cell, which comprises the vector of the fouth aspect of the present invention or the polynucleotide of the third aspect of the present invention integrated in the genome.

In the sixth aspect of the present invention, the present invention provides an immunoconjugate, which includes:
(a) an PD-L1 antibody or its active fragment of the first aspect of the present invention; and
(b) a coupling moietyselected from the following group consisting of a detectable label, a drug, a toxin, a cytokine, a radionuclide, or an enzyme, or a combination thereof.

In the seventh aspect, the present invention provides a pharmaceutical composition, which comprises:
(a) the PD-L1 antibody or its active fragment of the first aspect of the present invention, or the recombinant protein of the second aspect of the present invention, or the immunoconjugate of the sixth aspect of the present invention; and
(b) a pharmaceutically acceptable expression vector.

In another preferred embodiment, the pharmaceutical composition also comprises additional active components, preferably the active components comprises a small molecule chemical compound, a cytokine, or an antibody (such as anti-PD-1 antibody, anti-OX40 antibody, anti-CD137 antibody, anti-CD47 antibody, ADC, and CAR immunocells), or a combination thereof.

In another preferred embodiment, the pharmaceutical composition is in an injectable formulation.

In another preferred embodiment, the pharmaceutical composition is used to prepare a medicine to treat cancer patients. The cancer is selected from the group comprsing bladder cancer, brain cancer such as glioma and low-grade glioma, glioblastoma, cervical cancer, breast cancer, colon cancer, rectal cancer, endometrial carcinoma, kidney cancer, renal cell carcinoma, carcinoma of renal pelvis, leukemia, lung cancer, non small cell lung cancer (NSCLC), melanoma, non hodgkin's lymphoma, pancreatic cancer, prostate cancer, ovarian cancer, fibrosarcoma, acute lymphoblastic leukemia (ALL), acute myeloid leukemia (AML), chronic lymphoblastic leukemia (CLL), chronic myelogenous leukemia (CML), multiple myeloma, bone narrow derived tumor, thyroid cancer.

In another preferred embodiment, the pharmaceutical composition can be used to prepare a medicine for treating infection diseases, the infection diseases comprise viral infection diseases, bacterial infection diseases, fungal infection diseases.

In another preferred embodiment, in the infection diseases, cells at local infection or infected host cellsexpress PD-L1 (HIV, viral hepatitis, tuberculosis bacillus).

In another preferred embodiment, the viral infectious diseases are caused by pathogens selected from the group consisting of: HIV, hepatitis virus (a, b, c), herpes virus, adenovirus, influenza virus, arborvirus, Echovirus, Rhinovirus, Coxsackie viurs, coronavirus, respiratory cyncytial virus, mumps virus, rotaviurs, measles virus, Rubella virus, parvovirus, Vaccinia virus, human T lymphotropic virus, Dengue virus, Papilloma virus, soft wart virus, Poliovirus, rabies virus, Japanese encephalitis virus, arbovirus and encephalitis virus.

In another preferred embodiment, the bacterial infection diseases are caused by pathogens selected from the group consisting of: tuberculosis bacillus, chlamydia, Rickettsia, staphylococcus, streptococcus, Pneumococcus, Meningococcus, Neisseria gonorrhoeae, klebsiella pneumoniae, Proteus, Serratia marcescens, pseudomonas, Legionella, Diphtheria bacilli, Salmonella, Bacillus, Vibrio cholerae, Bacillus tetanus, Botulinum, Bacillus anthracis, Yersinia pestis, Leptospira, and Borrelia bacterium.

In another preferred embodiment, the fungal contagious diseases are caused by pathogens selected from the group consisting of: Candida, Cryptococcus neoformans, Aspergillus, Mucor, Sporothrix schenckii, Blastomyces dermatitis, Paracoccidium brasiliensis, Coccidiospore, and Histoplasma capsulatum. In another preferred embodimet, the contagious diseases are caused by pathogens selected from the group consisting of: HIV, hepatitis virus, influenza virus, herpes virus, Giardia, plasmodium, Leishmania, Staphylococcus aureus, and Pseudomonas aeruginosa.

In another preferred embodimet, the infection diseases are caused by pathogens selected infection diseases: HIV, hepatitis virus (a, b, c), and tuberculosis bacillus.

In another preferred embodiment, the pharmaceutical composition can be used in combination with a tumor immunotherapy that comprises, but not limited to: a chemotherapy cytotoxic agent, an antibody of CD20, LAG-3, CD73, CD47, DLL3, FR, CTLA-4, OX40, CD137, or PD-1, and other anti-tumor immunoagents, an anti-angiogenic agent, a radiation therapy, an antibody drug conjugate (ADC), a targeting therapeutic agent or another anti-tumor agents thereof.

In another preferred embodiment, the pharmaceutical composition targets PD-L1 on the surface of cancer cells of a subject in need or enhancing the ADCC/CDCeffect, including adiministrating the pharmaceutical composition of the seventh aspect of the present invention to a subject in need.

In the eighth aspect of the present invention, the present invention provides a method for detecting PD-L1 protein in samples, comprising the steps of:
(1) samples obtained from subjects;
(2) samples are interacted with the PD-L1 antibody or its active fragment according to the first aspect of the invention;
(3) detemining PD-L1 levels in subjects.

In another preferred embodiment, the method is non-therapeutic and non-diagnostic.

In another preferred embodiment, the method is used for detection *in vitro.*

In the nineth aspect of the present invention, the present invention provides a kit for detecting PD-L1, the kit consists of one or more selected from the group, such as the PD-L1 antibody or its active fragment according to the first aspect of the invention, or the immunoconjugate of the sixth aspect of the invention, or the pharmaceutical composition of the seventh aspect of the invention, and its instruction manual.

In another preferred embodiment, the instructions are stated that the kit is used for detecting PD-L1 expressions in the subjects.

In another preferred embodiment, the kit is used to detect PD-L1 expression (i.e. PD-L1 positive) in tumors.

In the tenth aspect of the present invention, the present invention provides an immune cell, the antibody according to the first aspect of the invention is expressed by the immune cell or exposed on extracellular membrane surface of the cell.

In another preferred embodiment, the immune cell comprises NK cells, T cells.

In another preferred embodiment, the immune cell is from human or non-human mammalian species (such as murine).

In the eleventh aspect of the present invention, the present invention provides a method for treating a disease associated with abnormal PD-L1 expression or function, wherein the method comprises administering the pharmaceutically effective amount of the PD-L1 antibody according to the first aspect of the invention , the immunoconjugate of the sixth aspect of the invention, or the pharmaceutical composition of the seventh aspect of the invention, the immune cells of the tenth aspect of the invention, or other combinations thereof, to a subject in need.

In another preferred embodiment, the disease associated with abnormal PD-L1 expression or function is cancers or infection diseases.

In another preferred embodiment, the antibody can be used alone, or in combination with other therapeutic agents.

In another preferred embodiment, the other therapeutic agents comprise immune check-point blockers, adjuvants or vaccines.

It should be understood that within the scope of the present invention, each technical features of the present invention described above and the technical features in the following (as examples) may be combined with each other to form a new or preferred technical solution, which is not listed here due to space limitations.

### Description of Drawings

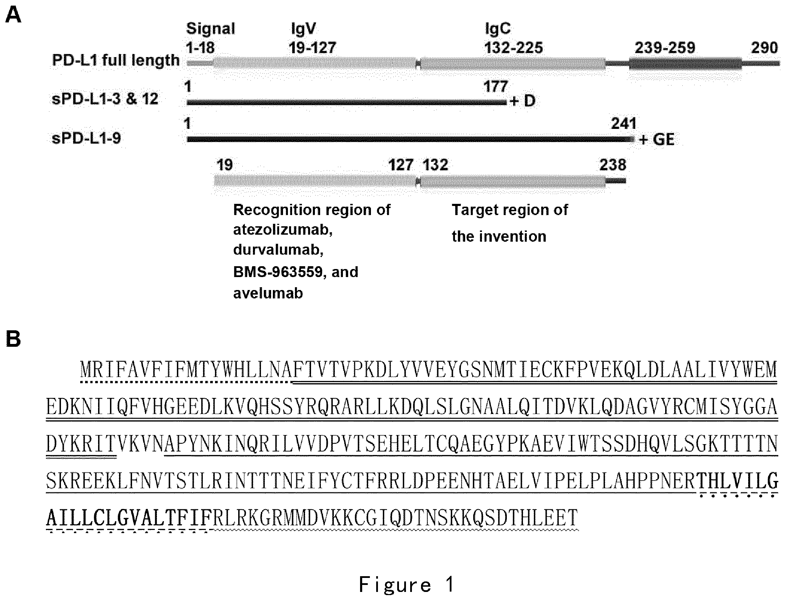

Figure 1. A. Full length of amino acid region of PD-L1 and its variants of sPD-L1-3 or 12 and sPD-L1-9, recognition regions of known PD-L1 antibodies and antigenic epitope of PD-L1 mouse monoclonal antibody mAb #19. B. Amino acid sequence of PD-L1 and IgC with a connection segment to cell membrane. Wherein a dotted underline indicates peptide signal, a double underline indicates IgV domain (amino acids 19-127), an underline indicates IgC and its connction segment to cell membrane (amino acids 132-238), a dotted and dashed underline indicates transmembrane domain (amino acids 239-259), a wavy underline indicates intracellular domain (amino acids 260-290).

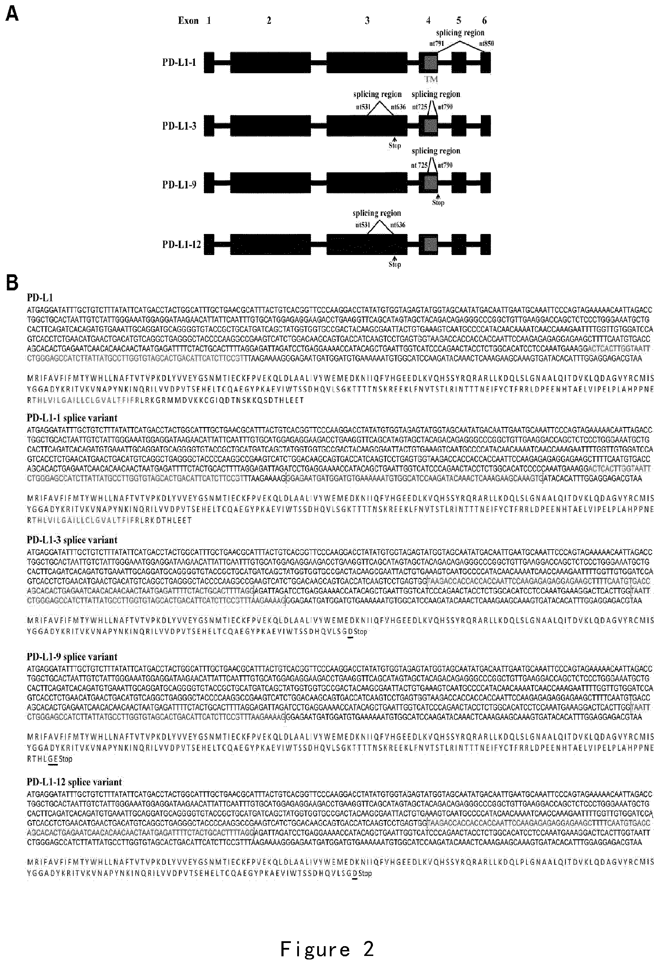

Figure 2. Schematic diagram of identified splice variants of PD-L1. Wherein, A. Full-length of PD-L1 comprising six exons. PD-L1-1, PD-L1-3, PD-L1-9, and PD-L1-12 are splicing variants, respectively. The transmembrane domain (TM) is located in exon 4 region and marked in pink. Spliced-out regions of PD-L1-1, 3, 9, 12 are indicated with bracket symbols. Stop represents a stop codon. B. Full length of nucleotide and amino acid sequences of membrane-bound PD-L1 and splice variants. Wherein the transmembrane domain is located within exon 4 region and shown in pink, spliced-out regions are indicated with bracket symbols and shown in red, each amino acid sequence of the identified splice variants is shown in blue, underline indicates additional different amino acids.

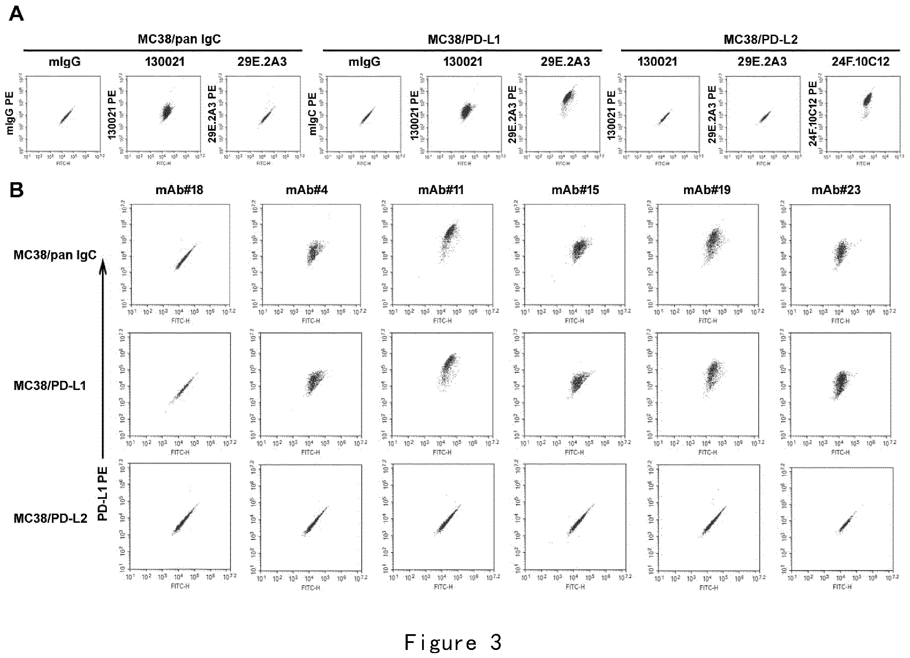

Figure 3. Binding of PD-L1 mouse monoclonal antibody to human PD-L1 and pan IgC. A. Binding of PD-L1 mouse monoclonal antibody #130021 (R&D systems, USA) and #29E.2A3 (Biolegend, USA) to either human PD-L1 or pan IgC was examined by flow cytometry. Wherein, PD-L1 mouse monoclonal antibody #130021 only binds to pan IgC, PD-L1 mouse monoclonal antibody #29E.2A3 only binds to PD-L1 but not pan IgC, indicating its recognition region is located in IgV, #24F.10C12 is a human PD-L2 mouse monoclonal antibody (Biolegend). B. PD-L1 mouse monoclonal antibodies were screened by flow cytometry. Wherein, five of PD-L1 mouse monoclonal antibody mAb#4, mAb#11, mAb#15, mAb#19, and mAb#23 were screened out to recognize both PD-L1 and pan IgC. Wherein, PD-L1 PE represents PE fluorochrome labeled PD-L1 antibody.

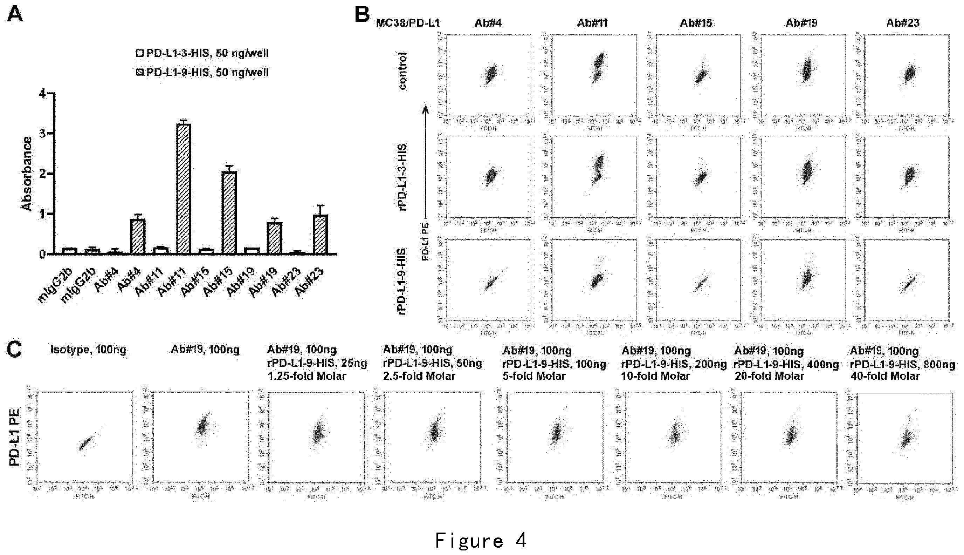

Figure 4. Affinity of PD-L1 mouse monoclonal antibody to rPD-L1 recombinant protein. A. Affinity of five PD-L1 mouse monoclonal antibodies of mAb#4, mAb#11, mAb#15, mAb#19, and mAb#23 to PD-L1 recombinant protein was examined by ELISA. B. Blocking effects of rPD-L1-3 and rPD-L1-9 recombinant proteins on binding of five PD-L1 mouse monoclonal antibodies of mAb#4, mAb#11, mAb#15, mAb#19, and mAb#23 to PD-L1 were examined by flow cytometry. C. Impacts of rPD-L1-9-HIS at different concentrations on binding of mouse monoclonal antibody #19 to PD-L1 were examined by flow cytometry.

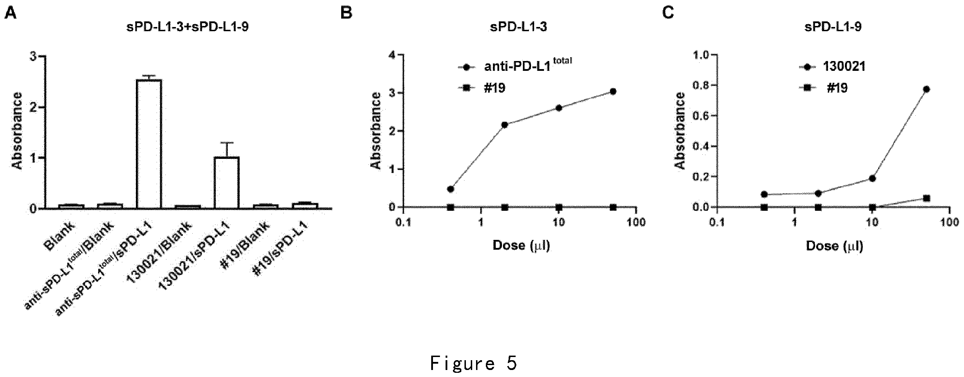

Figure 5. Affinity of PD-L1 mouse monoclonal antibody #19 to native forms of sPD-L1 examined by ELISA. A. Affinity of mouse monoclonal antibody mAb#19 to sPD-L1-3 and sPD-L1-9 in cell culture supernatant, B. Affinity of mouse monoclonal antibody mAb#19 to sPD-L1-3 in cell culture supernatant. C. Affinity of mouse monoclonal antibody mAb#19 to sPD-L1-9 in cell culture supernatant. Wherein, blank represent culture medium as a negative control.

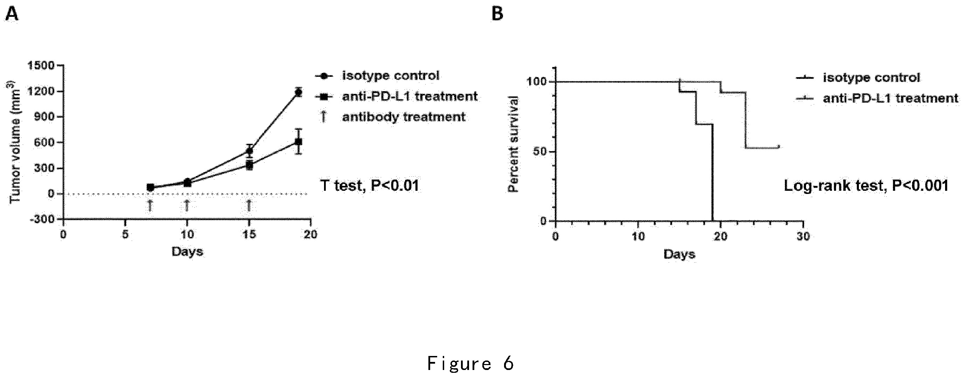

Figure 6. Anti-tumor effect of PD-L1 mouse monoclonal antibody #19 on human PD-L1 and sPD-L1-9-expressed MC38 tumor cells in mouse tumor model. A. Tumor sizes versus different mouse growth days, indicated as average tumor volume ± standard error. B. Survival rates versus different mouse growth days.

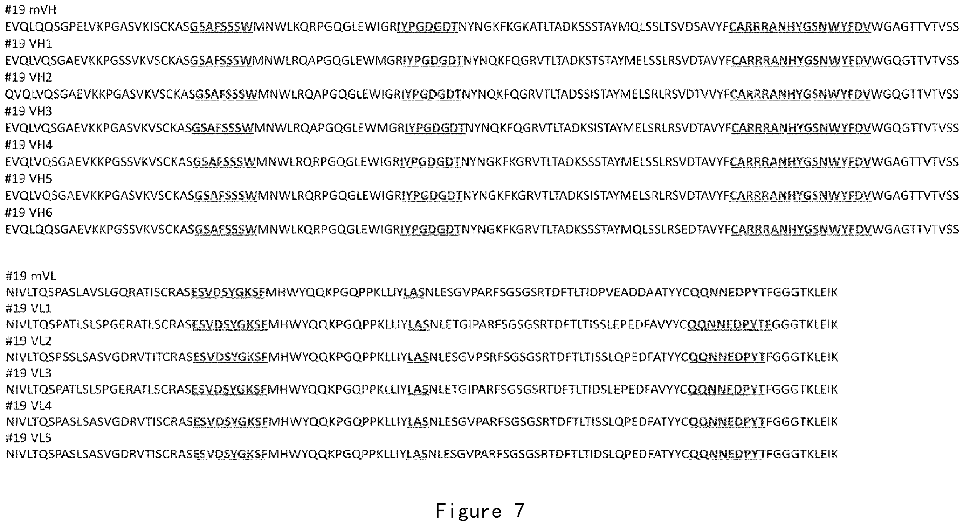

Figure 7. The humanized mouse monoclonal antibody #19. The Fab amino acid sequences of the different heavy(A) and light(B) chains are shown herein. mVH and mVL: the heavy-chain and light-chain variable regions of the mouse monoclonal antibody. VH and VL: the heavy-chain and light-chain variable regions of the humanized antibody. CDRs of heavy chain and light chain is indicated in red bold and underlined.

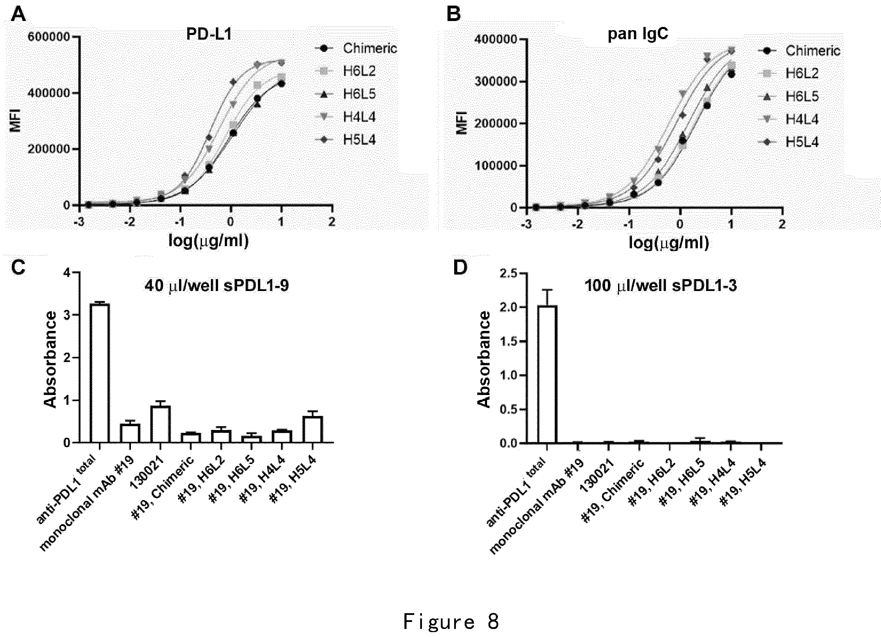

Figure 8. Affinity of the combinations from the different heavy (H) and light (L) chains of humanized mouse monoclonal antibody #19 to membrane-bound types PD-L1, secretory types of sPD-L1, and pan IgC.

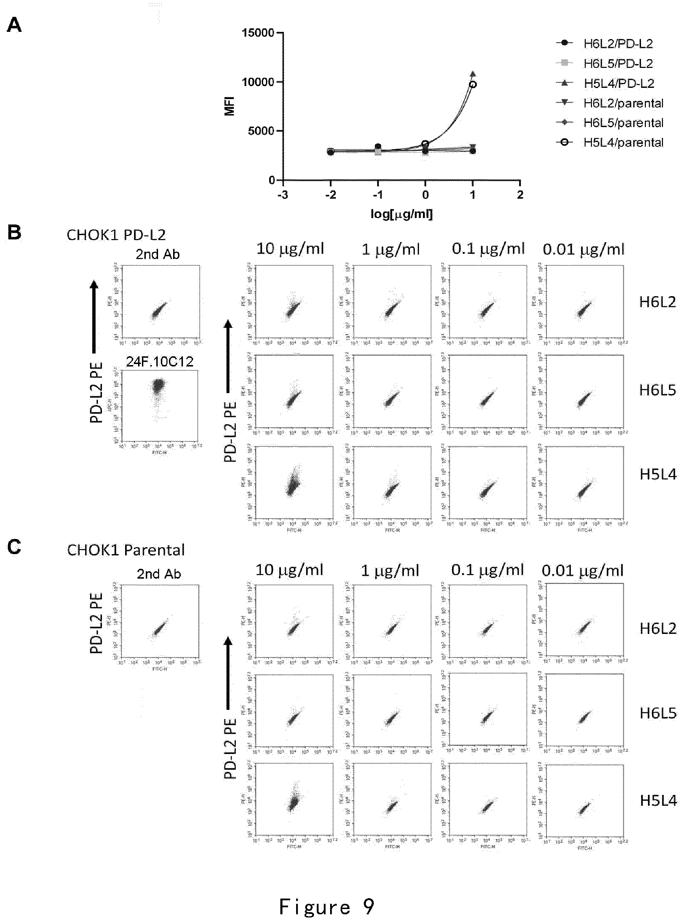

Figure 9. Affinity of the combinations from the different heavy (H) and light (L) chains of humanized mouse monoclonal antibody #19to membrane-bound type PD-L2 (human). Affinity of humanized mouse monoclonal antibody #19 to membrane-bound type PD-L2 was detected by flow cytometry (A. MFI plot; B. dot plot). C. CHO-K1 parental cells were used as a negative control. Clone #24F. 10C12: human PD-L2 mouse monoclonal antibody. 2^{nd} antibody: PE labeled goat anti-mouse antibody (BD Biosciences, USA)

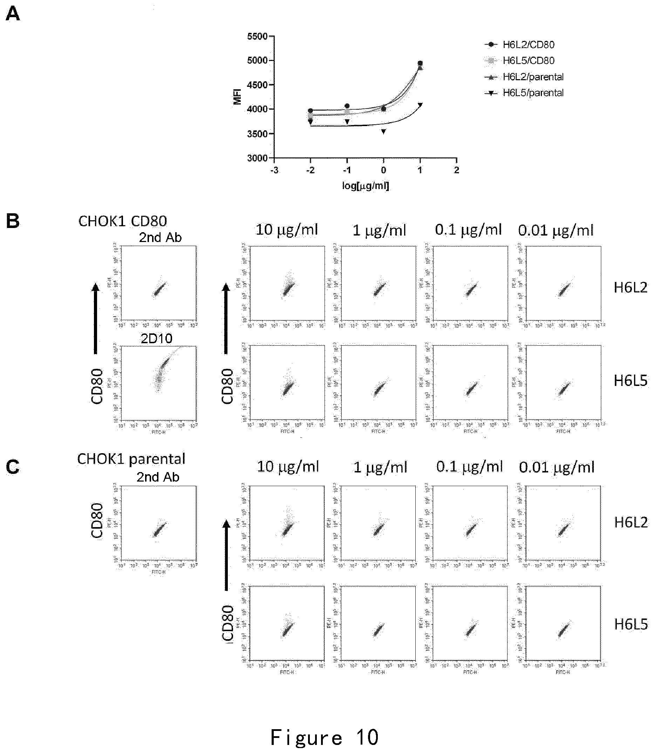

Figure 10. Affinity of the combinations from the different heavy (H) and light (L) chains of humanized mouse monoclonal antibody #19 to membrane-bound type CD80 (human). Affinity of humanized mouse monoclonal antibody #19 to membrane-bound type CD80 (human) was detected by flow cytometry (A. MFI plot; B. dot plot). C. CHO-K1 parental cells were used as a negative control. Clone #2D10: human CD80 mouse monoclonal antibody (Elabscience, Wuhan). 2^{nd} antibody: PE labeled goat anti-mouse antibody (BD Biosciences)

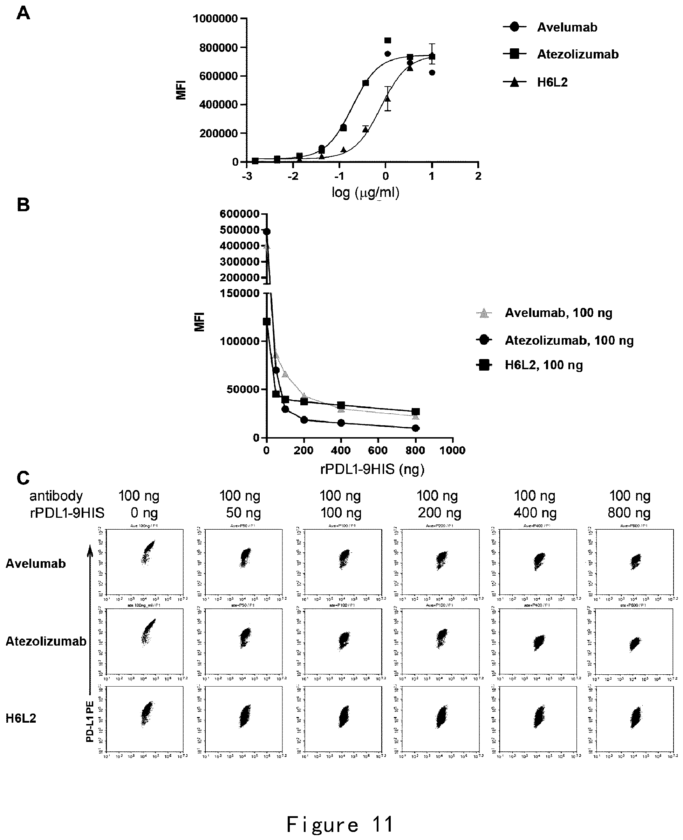

Figure 11. Affinity of humanized antibody H6L2 to membrane-bound type PD-L1 in presence or absence of rPD-L1-9-HIS. Affinity of humanized antibody H6L2 to membrane-bound type PD-L1 (A) and impact of rPD-L1-9-HIS on the affinity (B, MFI plot; and C, dot plot) were examined by flow cytometry, in comparison with avelumab and atezolizumab.

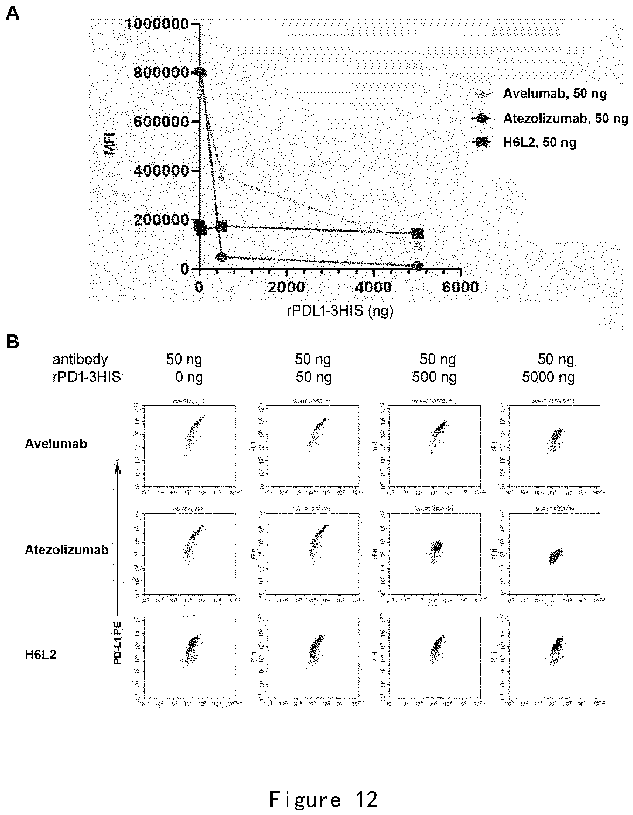

Figure 12. Affinity of humanized antibody H6L2 to membrane-bound PD-L1 in presence or absence of rPD-L1-3-HIS. Affinity of humanized antibody H6L2 to membrane-bound PD-L1 (A) and impact of rPD-L1-3-HIS on the affinity (B, MFI plot; and C, dot plot) were examined by flow cytometry, also in comparison with avelumab and atezolizumab.
Figure 13. Blocking effect of humanized antibody H6L2 on binding of PD-1 to PD-L1.

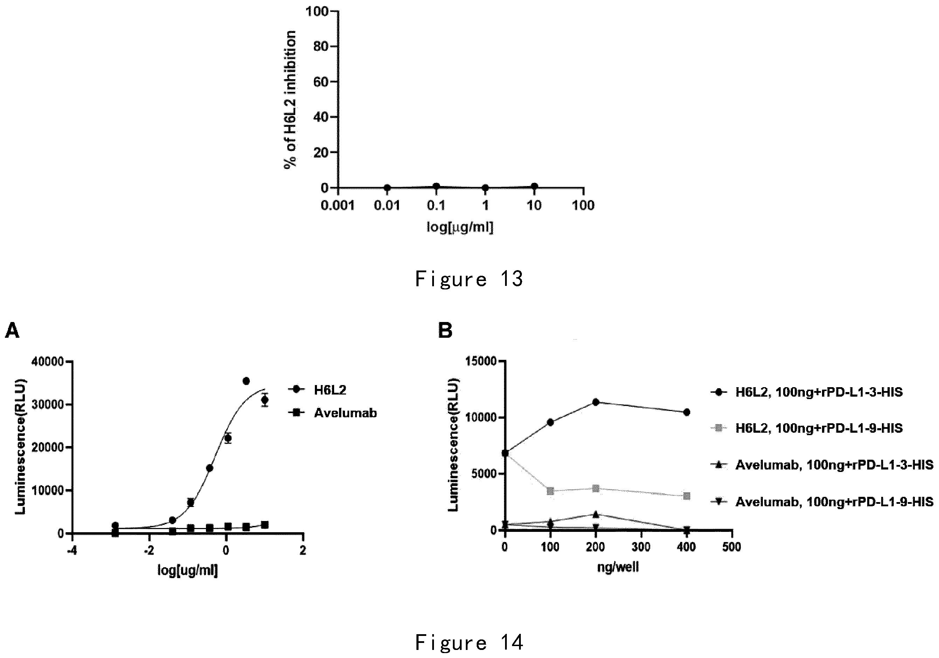

Figure 14. ADCC activity of humanized antibody H6L2, and impact of sPD-L1 on its ADCC. Humanized antibody #19 induced Luciferase activities in absence (A) or presence (B) of sPD-L1 (recombinant protein PD-L1-3-HIS or sPD-L1-9-HIS) were examined by Jurkat-CD16-NFAT-Luciferase system, also in comparison with avelumab.

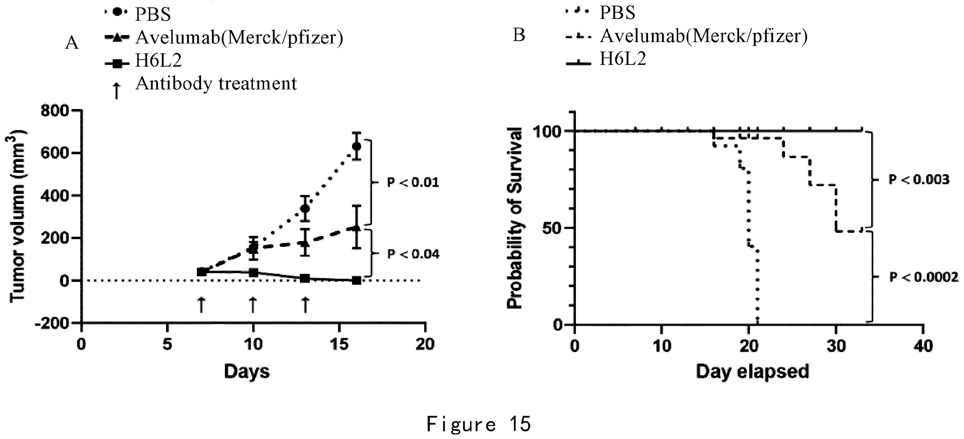

Figure 15. Anti-tumor effect of humanized antibody H6L2. A. Tumor size, average tumor volume of each group ± standard error. B. Survival rate. PBS and avelumab were used as negative and positive group, respectively.

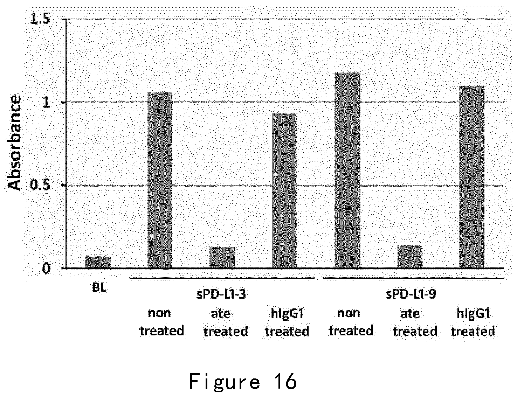

Figure 16. Binding of human PD-L1 antibody atezolizumab to native forms of sPD-L1. Binding of human PD-L1 antibody atezolizumab to native forms of sPD-L1 were examined by ELISA. Wherein, BL indicates supernatant without sPD-L1 used as background control; ate represents atezolizumab; hIgG1 treated represents human IgG1 treated; culture supernatant containing either sPD-L1-3 or sPD-L1-9 was indicated by sPD-L1-3 or sPD-L1-9, respectively.

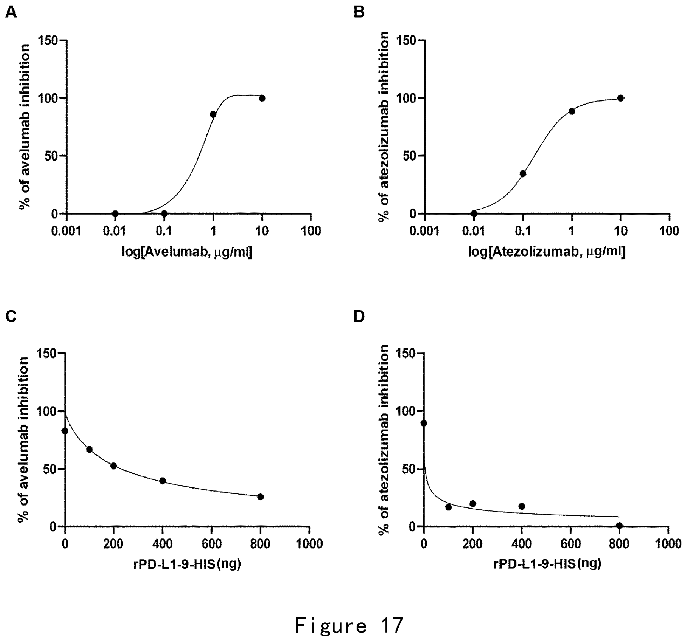

Figure 17. Blocking effects of human PD-L1 antibody avelumab and atezolizumab on binding of PD-1 to PD-L1, and impact of sPD-L1 on their blocking effects. A and B. Impacts of avelumab and atezolizumab on intercellular interaction of PD-1 with PD-L1. C and D. Impacts of sPD-L1 on blockade of avelumab and atezoliumab. Antibody concentration is 100 ng/datum point, rPD-L1-9-HIS concentration is shown in the Figure.

### Modes for Carrying Out the Present Invention

The inventor for the first time discovered a class of non-blocking antibodies by broad and in-depth study. The antibodies do not block the binding of PD-L1 to PD-1. However, the non-blocking PD-L1 antibody actually shows excellent activity against tumors. The study shows that the non-blocking antibody of the invention specifically targets pan IgC segment of PD-L1 and circumvents sPD-L1 to the most extent and in the meanwhile induces ADCC/CDC activity. The present invention has been completed on the basis of these studies.

In details, the inventor generated five specific anti-human PD-L1 mouse monoclonal antibodies that don't bind to sPD-L1-3, and one of them is able to partially circumvent sPD-L1-9, a full length of extracellular domain of PD-L1. In this respect, the humanized antibodies thereof are significantly superior to the control anti-human PD-L1 atezolizumab and avelumab antibodies.

### Term

In order to better understand disclosure of the invention, some terms need definitions firstly. Unless otherwise specified herein, each of following terms shall have the meaning given below. Other definitions are set forth throughout the application.

In the invention, term "non-blocking antibody of the invention", "PD-L1 antibody of the invention", "specific antibody of the invention", "antibody of the invention", "protein of the invention", or "polypeptide of the invention" can be used interchangeably, all of them refer to antibody that specifically binding to PD-L1. The non-blocking PD-L1 antibody of the invention doesn't block the binding of PD-L1 to PD-1 and specifically target pan IgC segment of PD-L1 and possess ADCC/CDC activity. For example, protein and/or polypeptide comprise heavy chain (such as amino acid sequences as shown in SEQ ID NO:11, 20, 21, 22, 23, 24, and 25) and/or light chain (such as amino acid sequence as shown in SEQ ID NO:15, 26, 27, 28, 29 and 30). They comprise or don't comprise methionine as a starting amino acid.

### pan IgC segment

As used herein, IgC domain and a connection segment to cell membrane are distinguished and referred as "pan IgC segment", a segment of amino acids 132-238 of PD-L1 protein SEQ ID NO: 19 (A and B from Figure 1).

### PD-L1 antibody

The invention provides PD-L1 antibody with high affinity to human PD-L1 protein. The tested antibody exhibits the effective binding and the anti-tumor activity and can be applied to therapeutic and diagnostic use. PD-L1 is a 40kDa type I transmembrane protein. Its extracellular segment comprises N-terminal immunoglobulin V domain (IgV) (amino acids 19-127) and C-terminal immunoglobulin C (IgC) domain (amino acids 132-225). As the interaction of antibody IgV domain with T cell receptor, PD-1 shows conserved front and side interactions of its IgV domain with PD-L1. The current PD-L1 antibodies all bind to the IgV domain, thereby blocking the binding of PD-L 1 to PD-1 (A and B of Figure 1). Therefore, the invention reveals a discovery: e.g., after binding to pan IgC segment, the non-blocking PD-L1 antibody of the invention effectively utilizes PD-L1 target leading to further improvement of therapeutic effect. In one embodiment of the invention disclosure, a PD-L1 antibody or its active fragments is provided, which specifically binds to pan immunoglobulin C (IgC) segment of human programmed death-ligand1 (PD-L1) protein. The antibody of the invention possesses one or more of expected functional features including, but not limited to high affinity to PD-L1, high specificity to PD-L1, induction of complement dependent cytotoxicity (CDC), antibody dependent phagocytosis (ADPC) and/or antibody-dependent cell-mediated cytotoxicity (ADCC) against the cells expressing PD-L1, and the antibody has the ability to inhibit tumor growth in single use or in combination therapy in subjects and animal models.

In some embodiment, pan IgV segment consists of amino acid residues 132-238. In some embodiment, the antibody or its active fragment can bind to the amino acid residues of PD-L1.

In one preferred embodiment of the present invention, the PD-L1 amino acid sequence consists of:

MRIFAVFIFMTYWHLLNAFTVTVPKDLYVVEYGSNMTIECKFPVEKQLDLAALIVY
WEMEDKNIIQFVHGEEDLKVQHSSYRQRARLLKDQLSLGNAALQITDVKLQDAGVYR
CMISYGGADYKRITVKVNAPYNKINQRILVVDPVTSEHELTCQAEGYPKAEVIWTSS
DHQVLSGKTTTTNSKREEKLFNVTSTLRINTTTNEIFYCTFRRLDPEENHTAELVIP
ELPLAHPPNERTHLVILGAILLCLGVALTFIFRLRKGRMMDVKKCGIQDTNSKKQSDT
HLEET (SEQ ID NO:19).

Wherein, positions 132-238 of SEQ ID NO:19 is pan IgC segment.

MRIFAVFIFMTYWHLLNAFTVTVPKDLYVVEYGSNMTIECKFPVEKQLDLAALIVY
WEMEDKNIIQFVHGEEDLKVQHSSYRQRARLLKDQLSLGNAALQITDVKLQDAGVYRCMI
SYGGADYKRITVKVNAPYNKINQRILVVDPVTSEHELTCQAEGYPKAEVIWTSSDHQVLS
GKTTTTNSKREEKLFNVTSTLRINTTTNEIFYCTFRRLDPEENHTAELVIPELPLAHPPN
ERTHLVILGAILLCLGVALTFIFRLRKGRMMDVKKCGIQDTNSKKQSDTHLEET
(SEQ ID No: 19)

PD-L1 amino acid segments and their positions as shown in the table below.

| Segments | Position in SEQ ID NO:19 | Note |
|---|---|---|
| Signal peptide | 1-18 | Based on labeled positions in SEQ ID NO:19 |
| IgV domain | 19-127 | |
| IgC domain | 132-225 | |
| A connection segment between IgC and cell membrane(pan IgC region) | 132-238 | |
| | Wherein, IgC: 132-225 | |
| Transmembrane domain | 239-259 | |
| Intracellular domain | 260-290 | |

### Secretorytype of sPD-L1 splicing variants

The inventor for the first time discovered secretory type of PD-L1 splicing variants that are associated with secretion of sPD-L1. sPD-L1 secretion is proportional to membrane-bound PD-L1 expression, and also associated with PD-L1 splicing activity, cytokine stimulations, and cell injury and death. The sequences of human PD-L1 splicing variants of PD-L1-1, PD-L1-3, PD-L1-9 and PD-L1-12 are shown in Figure 2 (Note: amino acid sequence of PD-L1-12 is identical to PD-L1-3). Wherein, "Secretory type of PD-L1", "Soluble PD-L1", "Secretion form of sPD-L1", and "sPD-L1" can be used interchangeably, representing secretion types of PD-L1 splicing variants.

In one specific embodiment, native forms of secretion types of sPD-L1 are expressed and secreted from MC38 cells; rPD-L1-HIS is secretory type sPD-L1 with HIS tagged that expressed and secreted by HEK293 cells, including PD-L1-3-HIS and PD-L1-9-HIS.

In another preferred embodiment, five PD-L1 specific antibodies are successfully selected, and they recognize both PD-L1 and IgC that expressed on cells. The five antibodies don't bind to sPD-L1-3, and one of them partially circumvents sPD-L1-9, which has full length of extracellular region comprising IgV and IgC domains. *In vivo* experiment in sPD-L1-9 secretion mice tumor model, the antibody showed anti-resistance and anti-tumor effects.

In another preferred embodiment, PD-L1 mouse monoclonal antibody #19 was selected.

Antibody #130021 (a control antibody), e.g., a mouse monoclonal antibody, (R&D systems), binds to pan IgC segment of PD-L1, and do not bind to sPD-Ll-3 in soluble PD-L1 protein, but bind to sPD-Ll-9.

As used herein, the term "antibody" or "immunoglobulin" is a heterotetrameric glycoprotein of about 150000 Daltons, which consist of two identical light chains (L) and two identical heavy chains (H). Each light chain is linked to a heavy chain by a covalent disulfide bond, while the number of disulfide bonds varies among the heavy chains of different immunoglobulin isotypes. Each heavy and light chain also has regularly spaced intrachain disulfide bridges. Each heavy chain has at one end a variable domain (VH) followed by constant domains. Each light chain also has a variable domain (VL) at one end and a constant domain at its other end. The constant domain of the light chain is aligned with the first constant domain of heavy chain, and the variable domain of light chain is aligned with the variable domain of the heavy chain. Particular amino acid residues form an interface between light-chain and heavy-chain variable domains.

As used herein, the term "variable" means that certain portions of the variable domains of antibody differ extensively in sequences, which results in affinity and specificity of each particular antibody to its particular antigen. However, the variability is not evenly distributed throughout the entire variable domains of antibodies. It is concentrated in three segments called Complementarity Determining Regions (CDRs) or hypervariable regions in the light and heavy chain variable region. The more highly conserved portions of the variable regions are called Framework Regions (FR). The variable regions of native heavy and light chains each comprise four FR regions, in a substantially β- sheet configuration, connected by three CDRs that form a linking loop, and in some cases may form a part of the β- sheet structure. The CDRs in each chain are closely linked together by the FR regions and form the antigen binding site of the antibodies with the CDRs from the other chain (see Kabat et al, NIH Publ. No.91-3242, Vol I, 647-669 (1991)). The constant regions are not directly involved in binding of antibodies to antigens, but they exhibit various effector functions, such as participation of the antibody in antibody-dependent cytotoxicity.

The light chain of antibody (immunoglobulin) from any vertebrate species can be assigned to one of two clearly distinct types (called λ and κ), based on the significant differences in amino acid sequences of their constant regions. Also, depending on amino acid sequences of heavy chain constant regions, immunoglobulins can be assigned to different classes. There are five major classes of immunoglobulins: IgM, IgD, IgG, IgA and IgE, some of them can be further divided into subclasses (isotypes), such as IgG1, IgG2, IgG3, IgG4, IgA1 and IgA2. The heavy chain constant regions corresponding to different classes of immunoglobulins are called α, β, ε, γ, and µ, respectively. Subunit structure and three-dimensional configuration of different classes of immunoglobulins are well-known to those in the art.

As used herein, the term "monoclonal antibody (mAb)" refers to an antibody obtained from substantially homogeneous population, i.g. the individual antibodies comprising the population are identical except for possible naturally occurring mutations that may be present in minor amounts. Monoclonal antibody is highly specifically directed against a single antigenic site. Furthermore, in contrast to conventional polyclonal antibody preparations (In general, it refers to different antibodies targets distinct antigenic determinants), each monoclonal antibody targets one single determinant on the antigen. Besides their specificity, monoclonal antibody has advantages of being homogeneously synthesized by hybridoma culture, uncontaminated by other immunoglobulins. The modifier "Monoclonal" indicates the character of antibody as being obtained from a substantially homogeneous population of antibodies, and is not to be construed as requiring production of the antibody by any particular method.

The invention also includes the corresponding amino acid sequence of monoclonal antibody to PD-L1 protein, the variable region chains of the monoclonal antibody against PD-L1 protein, and the other proteins or protein conjugate and fusion expression product thereof, which comprise the variable region chains. Specifically, the invention includes any protein or protein conjugate and fusion expression product (i.g. immunoconjugate and fusion expression product), which comprise hypervariable regions (Complementarity Determining Regions, CDR) of light chain and heary chain, provided that the hypervariable regions are identical or at least 90% homologous, or preferably at least 95% homologous to the light chain and heavy chain of the invention.

As known to persons skilled in the art, immunoconjugate and fusion expression product include: a drug, a toxin, a cytokine, a radionuclide, an enzyme and other diagnostic or therapeutic molecules, and a conjugate formed with the monoclonal antibody or its fragment thereof against PD-L1 protein. The invention also includes biomarkers or antigens on surface of cells, to which the monoclonal antibody or its fragment against PD-L1 protein thereof binds.

The invention includes not only an intact monoclonal antibody but also immune active antibody fragments such as Fab or (Fab')₂ fragment; antibody heavy chain; antibody light chain.

As used herein, the term "Heavy chain variable region" and "VH" can be used interchangeably.

As used herein, the term "variable region" and "CDR (complementarily determining region)" can be used interchangeably.

In another preferred embodiment, heavy-chain variable region of the antibody includes three complementarily determining regions (CDR1, CDR2, and CDR3).

In another preferred embodiment, the heavy chain of the antibody comprises heavy chain variable and heavy chain constant regions as described above, the heavy chain constant domain may derived from murine or human species.

In another preferred embodiment, the antibody is a mouse or human-mouse chimeric monoclonal antibody against PD-L1 protein, its heavy-chain constant region and/or light-chain constant region may be a humanized constant region of heavy chain or a humanized constant region of light-chain. More preferably, the humanized constant region of heavy chain and the humanized constant region of light chain may be heavy chain constant region or light chain constant region of human IgG1, IgG2, etc.

The invention also provides other proteins or fusion expression product of the antibody of the present invention. Specifically, the invention includes any of protein or protein conjugate and fusion expression product (i.e., immunoconjugate and fusion expression product), which comprises heavy chain and light chain regions, provided that the variable regions are identical or at least 90% homologous, preferably at least 95% homologous to the light chain and heavy chain of the invention.

In general, antigen binding properties of antibody may be described by three specific regions located in the variable regions of the heavy chain and light chain, called the variable regions (CDRs), which are separated into four framework regions (FRs), the amino acid sequences of the 4 FRs are relatively conserved and doesn't directly participate in the binding activity. The CDRs formed loop structures through β pleated sheet formed by the FRs between them are spatially close to each other, the CDRs on the heavy chain and the CDRs on the corresponding light chain consists of antigen binding site of antibody. In alignment with amino acid sequences of antibody isotypes, those amino acids for formation of FR and CDR region are determined.

The variable regions of the heavy and light chains in the invention are of particularly interest, because of at least parts of them involved in antigen binding. Therefore, the invention includes those molecules of monoclonal antibody light-chain and heavy-chain variable region, which comprise of CDR, provided that homology of their CDR to the CDR identified herein is greater than 90% (preferably greater than 95%, more preferably greater than 98%).

The invention includes not only an intact monoclonal antibodies but also an antibody fragment with immunological activity or a fusion protein formed with antibody and other sequences. Accordingly, the invention also includes fragment, derivative and analogue of the antibody.

As used herein, the terms "fragment," "derivative," and "analogue" refer to a polypeptide that retains substantially the same biological function or activity as an antibody of the invention. A polypeptide fragment, derivative or analogue of the invention may be (i) a polypeptide in which one or more conserved or non-conserved amino acid residues (preferably conserved amino acid residues) are substituted, and such substituted amino acid residues may or may not be encoded by the genetic code, or (ii) a polypeptide having a substituent group in one or more amino acid residues, or (iii) a polypeptide in which the mature polypeptide is fused to another compound (such as a compound that extends the half-life of the polypeptide, e.g., polyethylene glycol), or (iv) a polypeptide in which an additional amino acid sequence is fused to the sequence of the polypeptide (e.g., a leader or secretory sequence or a sequence used to purify the polypeptide or a proprotein sequence, or a fusion protein with a 6His tag). Such fragments, derivatives and analogs are within the purview of persons skilled in the art in view of the teachings herein.

The antibody of the invention refers to the polypeptide that has binding activity to PD-L1 protein and comprises the CDR regions as described above. The terms also includes polypeptide variants that comprise CDR regions as described above and have the same function as the antibody of the invention. The variants include (but not limited to): one or more (ordinarily 1-50, preferably 1-30, more preferably 1-20, most preferably 1-10) of deletion, insertion and/or substitution of amino acids, and one or more of additional amino acids at C-terminus and/or N-terminus (ordinarily up to 20, preferably up to 10, more preferably up to 5). In the field, for example, protein functions generally would not be changed by replacements of amino acids with those whose properties are close or similar to the substitutes. In another example, protein functions generally would not be changed by addition of one or more amino acids at the C-terminus and/or N-terminus. The term also includes the active fragment and derivative of the antibody of the present invention.

Variants of the polypeptide include: homologous sequences, conservative variants, allelic variants, natural mutants, induced mutants, proteins encoded by DNA that hybridizes under high or low stringency conditions with DNA encoding the antibody of the invention, and polypeptides or proteins obtained using antisera raised against the antibody of the invention.

The invention also provides other polypeptides, such as fusion proteins comprising human antibodies or fragments thereof. In addition to nearly full-length of polypeptides, the antibody fragments of the invention are also encompassed by the present invention. Typically, the fragment has at least about 50 contiguous amino acids of the antibody of the invention, preferably at least about 50 contiguous amino acids, more preferably at least about 80 contiguous amino acids, and most preferably at least about 100 contiguous amino acids.

The invention also provides polynucleotide molecules encoding above described the antibody or its fragment, or the fusion proteins thereof. The polynucleotide of the present invention may be in the form of DNA or RNA. The form of DNA includes cDNA, genomic DNA or artificially synthesized DNA. The DNA may be single-stranded or double-stranded. The DNA may be the coding strand or the non-coding strand.

Polynucleotides encoding the mature polypeptides of the invention include: a coding sequence only encoding the mature polypeptide; the coding sequence for the mature polypeptide and various additional coding sequences; the coding sequence (and optionally additional coding sequences) as well as non-coding sequences for the mature polypeptide.

The term "polynucleotide encoding a polypeptide" may include a polynucleotide encoding the polypeptide, and may also include additional coding and/or non-coding sequences.

The present invention also relates to polynucleotides which hybridize to the sequences as described above and have at least 50%, preferably at least 70%, and more preferably at least 80% identity between the two sequences, and the present invention particularly relates to polynucleotides which hybridize to the polynucleotides of the present invention under stringent conditions, where "stringent conditions" refer to (1) hybridization and elution at lower ionic strength and higher temperatures, e.g., 0.2 × SSC, 0.1% SDS, 60 °C, or (2) hybridization with denaturing agents, e.g., 50% (v/v) formamide, 0.1% bovine serum/0.1% Ficoll, 42 °C, etc., or (3) hybridization only if the identity between the two sequences is at least 90% or more, and more preferably 95% or more, and the polypeptides encoded by the hybridizable polynucleotides have the same biological function and activity as the mature polypeptides.

The full-length nucleotide sequence of the antibody or its fragment of the present invention thereof can be obtained by a method of either PCR amplification or recombinant expression, or artificial synthesis. One possibility is to use a synthetic method synthesizing the sequence of interest, especially when the fragment length is short. Generally, fragments with long sequences are obtained by first synthesizing a plurality of small fragments and then ligating them. Alternatively, the coding sequence for the heavy chain and an expression tag (e.g., 6His) can be fused together to form a fusion protein.

Once the sequence of interest is obtained, it can be produced in large quantities by recombinant methods. This is usually done by cloning it into a vector, transferring it into a cell, and isolating the relevant sequence from the propagated host cell by conventional methods. The biomolecules (nucleic acids, proteins, etc.) to which the present invention relates include biomolecules in an isolated form.

At present, DNA sequences encoding the proteins of the present invention (or fragments or derivatives thereof) have been obtained completely by chemical synthesis. The DNA sequence may then be introduced into various existing DNA molecules (or vectors, for example) and cells known in the art. Furthermore, mutations can also be introduced into the protein sequences of the invention by chemical synthesis.

The invention also relates to a vector comprising a suitable DNA sequence as described above and a suitable promoter or control sequence. These vectors may be used to transform an appropriate host cell so that it can express the protein.

The host cells may be prokaryotic cells, such as bacterial cells; or lower eukaryotic cells, such as yeast cells; or higher eukaryotic cells, such as mammalian cells. Representative examples are: escherichia coli, streptomyces; bacterial cells of salmonella typhimurium; fungal cells such as yeast; insect cells of Drosophila S2 or Sf 9; animal cells of CHO, COS7, 293 cells, etc.

Transformation of a host cell with recombinant DNA can be carried out using conventional techniques well known to those skilled in the art. When the host is a prokaryote, e.g., E.coli, competent cells that can uptake DNA are harvested after exponential growth phase, which are generated using CaCl₂ Methods with the steps used well known in the art. Another method is to use MgCl₂. If necessary, transformation can also be carried out by electroporation. When the host is a eukaryote, the following DNA transfection methods may be used: calcium phosphate co-precipitation, conventional mechanical methods such as microinjection, electroporation, liposome encapsulation, and the like.

The obtained transformant can be cultured by a conventional method to express the polypeptide encoded by the gene of the present invention. The medium used in the culture may be selected from various conventional media depending on the host cell used. The culturing is performed under conditions suitable for growth of the host cell. After the host cells have been grown to an appropriate cell density, the selected promoter is induced by suitable means (e.g., temperature shift or chemical induction) and the cells are cultured for an additional period of time.

The recombinant polypeptides can be expressed intracellularly, or on the cell membrane, or secreted extracellularly by the methods as described above, if necessary, the recombinant proteins can be isolated and purified by various separation methods using their physical, chemical, and other properties, which are well known to those skilled in the art. These include but not limited to: methods of conventional renaturation process, protein precipitation solution (salting-out extraction), centrifugation, osmotic shock on bacteria, ultra-centrifugation, molecular sieve chromatography (gel filtration), adsorption chromatography, ion exchange chromatography, high performance liquid chromatography, and other various liquid chromatography analytical techniques and combinations of these methods.

The antibody of the invention may be used alone or in combination or conjugation with detectable labels (for diagnostic purposes), therapeutic agents, PK (protein kinase) modifying moieties or combinations of any of the above.

Detectable labels for diagnostic purposes include but are not limited to: a fluorescent or luminescent label, a radioactive label, an MRI (magnetic resonance imaging) or CT (computed tomography) contrast agent, or an enzyme capable of producing a detectable product.

Therapeutic agents that can be bind to or conjugated to the antibody of the invention include but are not limited to: 1. Radionuclides; 2. Biotoxins; 3. cytokines such as I L-2, etc.; 4. gold nanoparticles/nanorods; 5. viral particles; 6. Liposomes; 7. Nanomagnets; 8. prodrug activating enzymes [e.g., DT-diaphorase (DTD) or biphenyl hydrolase-like protein (BPH L)]; 9. chemotherapeutic agents (e.g., cisplatin) or any form of nanoparticles, etc.

### Immune conjugate

The invention provides immune conjugate, based on the antibody of the present invention, more preferably, the immune conjugate refers to as antibody-drug conjugate (ADC).

Typically, the antibody-drug conjugate (ADCs) comprises an antibody conjugated and an effector molecule, the antibody is conjugated to the effector molecule, and chemical coupling is preferred. Wherein, the effector molecules are preferred to be a drug with therapeutic activity. In addition, the effector molecule may be one or more of a toxic protein, a chemotherapeutic agent, a small molecule agent, or a radionuclide.

The antibody of the present invention couples to the effector molecule by a coupling agent. For example, the coupling agent may be any one or more of a non-selective coupling agent, a coupling agent using carboxyl group, a peptide chain, a coupling agent using disulfide bond. The non-selective coupling agent refers to chemical composition of effector molecule conjugated to antibody by covalent bond, such as glutaraldehyde etc. The coupling agent using carboxyl group may be any one or more of an aconitic anhydride like coupling agent (for example, cis-aconitic acid anhydride), or an acyl hydrazone like coupling agent (coupling site is acyl hydrazone).

Amino acid residues of antibody (for example, Cys or Lys, etc) are used to conjugate with groups with multiple functions, comprising imaging reagent (such as chromophores and flurophores), diagnostic reagent (such as MRI contrast agent and radioactive isotope), stabilizer (such as Ethylene glycol polymer) and therapeutic agent. Antibody may be conjugated (covalently linked) to functional agent in form of antibody-functional agent conjugate. Functional agent (such as drug, detection reagent, and stabilizer) is conjugated to antibody (by covalent bond linkage).

Antibody may be coupled to drug to form antibody drug conjugate (ADC). Typically, ADC comprises a linker between drug and antibody. Linker may be cleavable or non-cleavable. Typical degradable linker may be easily degraded in intracellular environment, for example, degradation reaction of linker occurs at target site, in order to release drug from antibody. Appropriate degradable linkers include, for example, enzymatic degraded linker, wherein, consisting of peptide linker that may be degraded by intracellular protease (such as lysosomal protease or *in vivo* protease), or sugar linker, such as glucuronide linker that may be degraded by glucuronidase. Peptide linkers include, for example, dipeptide, such as valine-citrulline, phenylalanine-lysine, or valine-alanine. Other appropriate degradable linkers include, for example, pH sensitive linker (such as pH < 5.5 cleavable linker, i.e., hydrazine linker) and cleavable linker under reducing condition (such as disulfide bond linker). Typical non-degradable linker releases drug under the condition that antibody is hydrolyzed by protease.

Drug may be any of cytotoxic agents, cell growth inhibitory agents or immunosuppression agents. In an example, a linker is connected to an antibody and a drug, and a drug has functional moieties that form bonds with linkers. For example, drug may have moieties of azyl, carboxyl, sulfydryl, hydroxyl, or keto, which can form connection bonds with a linker. When a dug isdirectly connected to a linker, a drug has reactive moieties before its bond to an antibody.

Classes of useful drugs include, for example, anti-microtubule agents, DNA groove binding reagents, DNA replication inhibitors, alkylating reagents, antibiotics, folic acid antagonists, antimetabolites, chemosensitizers, topoisomerase inhibitors, vinca alkaloids etc. Particularly useful cytotoxic drugs include, for example, DNA groove binding reagents, DNA alkylation reagents, and microtubule inhibitors. Typical cytotoxic agents include, for example, auristatins, camptothecins, duocarmycins, etoposides, maytansines, and maytansinoids (such as DM1 and DM4), taxane, benzodiazepines or benzodiazepine containing drugs (such as Pyrrolo [1,4] benzodiazepines (PBDs), indolinobenzodiazepines, and oxazolidinobenzodiazepines), and vinca alkaloids.

In the present invention, drug-linker is applied to form ADC via simple procedures. In another embodiment, bifunctional linker compound is applied to form ADC via two step or multiple step method. For example, cysteine residues react with reactive part of linker at the first step, functional moieties of linker subsequently react with drug to form ADC.

The present invention provides a method of ADC preparation that further includes: antibody are conjugated with drug-linker compound, under conditions sufficient to form antibody drug conjugate,.

In some examples, the present invention includes: under conditions sufficient to form antibody drug conjugate, antibody are conjugated with bifunctional linker compound. In these examples, the present invention further includes: under conditions sufficient on binding of drug part to antibody via covalent bond of linker, antibody-linker conjugate connects with drug part.

In certain examples, antibody-drug conjugate as shown in molecular formula below:

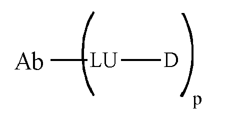

Wherein:
Ab represents antibody;
LU represents linker;
D represents drug;
and subscript p is a value selected from 1 to 8

### Pharmaceutical composition

The invention also provides a composition. Preferably, the composition is a pharmaceutical composition comprising the antibody or its active fragment or its fusion protein thereof as described above, and a pharmaceutically acceptable carrier. Generally, these materials will be formulated in a non-toxic, inert and pharmaceutically acceptable aqueous carrier medium, wherein the pH is generally from about 5 to about 8, preferably from about 6 to about 8, although the pH will vary depending on the nature of the material being formulated and the condition being treated. The formulated pharmaceutical compositions may be administered by conventional routes including, but not limited to: intratumoral, intraperitoneal, intravenous, or topical administration.

The pharmaceutical composition of the present invention can be used to bind directly to PD-L1 protein molecules and thus can be applied to prevention and therapy of cancer.

The pharmaceutical composition of the present invention comprises a safe and effective amount (e.g., 0.001-99 wt%, preferably 0.01-90 wt%, more preferably 0.1-80 wt%) of the monoclonal antibody (or its conjugate) of the present invention as described above and a pharmaceutically acceptable carrier or excipient. Such carriers include (but are not limited to): saline, buffer, glucose, water, glycerol, ethanol, and combinations thereof. The pharmaceutical preparation should be compatible with the mode of administration. The pharmaceutical composition of the present invention can be prepared in the form of an injection, for example, by a conventional method using physiological saline or an aqueous solution containing glucose and other adjuvants. Solution formulations are applied to pharmaceutical compositions such as injections and preferably manufactured under sterile conditions. The amount of active ingredient administered is a therapeutically effective amount, for example from about 1 microgram per kilogram of body weight to 5 milligrams per kilogram of body weight per day. In addition, the polypeptides of the present invention may also be used in combinations with other therapeutic agents.

In another specific example, pharmaceutical compositions also comprise additional active components, preferably including small molecules, cytokines, antibodies and others (e.g., anti-PD-1, anti-OX40, anti-CD137, and anti-CD47, ADC and CAR immune cells).

In another specific example, in treatment of cancers, inflammatory diseases, immune diseases, autoimmune diseases, and infection diseases, as well as their symptoms or conditions, pharmaceutical composition can be used in combination with another therapy, which includes but not limited to: chemotherapy, anti-CD20 mAb, anti-TIM-3 mAb, anti-LAG-3 mAb, anti-CD73 mAb, anti-CD47 mAb, anti-DLL3 mAb, anti-FR mAb, anti-CTLA-4 mAb, anti-OX40 mAb, anti-CD137 mAb, anti-PD-1 mAb, anti-PD-L1 mAb, and other anti-tumor immunoagents, anti-angiogenic agent, radiation therapy agent, antibody drug conjugate (ADC), targeting tumor therapeutic agent or other anti-tumor agents. A bispecific antibody may be co-constructed from a PD-L1 antibody and a partner antibody against cancer/tumor expressed and related antigens as following: PD-1, OX40, CD137, LAG3, TIM-3, CTLA-4, EGFR, HER-2, CD19, CD20, CD33, CD73, CD47, DLL-3, CLDN18.2, folic acid receptor-α(FOLR1), and/or tumor cell surface antigens.

In the case of pharmaceutical compositions, a safe and effective amount of the immunoconjugate is administered to the mammal, wherein the safe and effective amount is typically at least about 10 micrograms/kg body weight, and in most cases no more than about 8 milligrams/kg body weight, preferably the dose is from about 10 micrograms/kilogram body weight to about 1 milligram/kilogram body weight. Of course, the particular dosage will depend upon such factors as the route of administration, the health of the patient, and the like, and is within the skill of the skilled practitioner.

### Applications

Other therapies of the present invention are applied to patients exposed to certain toxins or pathogens. Therefore, in another aspect of the invention, an approach is provided for prevention and/or treatment of tumors or infectious diseases of objects, including the PD-L1 binding molecule of the present invention applied to the objects, and the objects receiving prevention and/or treatment of tumors or infectious diseases accordingly.

In another preferred embodiment, the pharmaceutical composition is in preparation for tumor therapeutic drug use, tumors are selected from the group below: bladder cancer, brain cancer such as glioma and low-grade glioma, glioblastoma, cervical cancer, breast cancer, colon cancer, rectal cancer, endometrial carcinoma, kidney cancer, renal cell carcinoma, carcinoma of renal pelvis, leukemia, lung cancer, non small cell lung cancer (NSCLC), melanoma, non hodgkin's lymphoma, pancreatic cancer, prostate cancer, ovarian cancer, fibrosarcoma, acute lymphoblastic leukemia (ALL), acute myeloid leukemia (AML), chronic lymphoblastic leukemia (CLL), chronic myelogenous leukemia (CML), multiple myeloma, bone narrow derived tumor, and thyroid cancer.

Similar to the applications to the above described tumors, the PD-L1 antibody of the present invention can be used alone, or used as an adjuvant in combination with a vaccine for stimulation of immune response to pathogens, toxins, and auto-antigens. In particular, the examples of pathogens treated by the therapy include pathogens without prevention of currently effective vaccine, or pathogens without complete prevention by conventional vaccine. Wherein, it includes, but not limited to HIV, hepatitis virus (A, B, C), influenza virus, herpes virus, Giardia, plasmodium, Leishmania, Staphylococcus aureus, and Pseudomonas aeruginosa. PD-L1 blocker is specifically applied to be against infectious diseases by pathogens such as HIV, antigen presentations are changed during infection. In human PD-L1 antibody administration, the neo-epitopes are regarded as exogenous identifiers, which induce robust T cell immune response without impact of PD-L 1 inhibitory signal.

Preferably, the antibody of the present invention is applied to treatment of infectious diseases. The infectious diseases treated by the antibody of the present invention are selected from the group below: HIV, viral hepatitis (A, B, C), tuberculosis bacillus.

In above described therapies, the antibody of the present invention can be used in combination with other immune therapies such as a cytokine (for example, GM-CSF, G-CSF, and IL-2) or bispecific antibody, bispecific antibody therapy can provide increased presentation of tumor antigen.

### Hybridoma

The invention also provides hybridoma for production of the monoclonal antibody of the present invention against PD-L1 protein; preferably, the invention provides the hybridoma that produces a high titer of monoclonal antibody against PD-L1 protein.

After hybridoma that produces the PD-L1 monoclonal antibody of the present invention is obtained, the antibody is easily prepared using the hybridoma cells by technician in the art. In addition, information on the antibody structure of the present invention (e.g., heavy chain and light chain variable regions) is also easily obtained by technician in the art, and the monoclonal antibody of the present invention is then manufactured by recombinant method.

### Methods and samples

The invention refers to methods on cell and/or tissue lytic samples for detection of tumors. The procedures are described as follows: obtaining cell and/or tissue samples; lysing the samples in a medium solution, detecting PD-L1 protein levels in the solution. In the detection method of the present invention, there are no any particular limitations on sample usage, for a representative example, cell-containing samples present in cell preservation solution.

### Kit

The invention also provides a reagent kit consisting of the antibody (or its fragments) of the present invention or the detection plate of the assay kit of the present invention, in a preferred embodiment of the invention, the reagent kit also includes containers, instructions for use, buffers, and the like.

The invention further designs a reagent kit applied to detect PD-L1 levels, which includes an antibody recognizing PD-L1 protein, a lytic medium for dissolving samples, general reagents and buffers required for the detection, such as various buffers, detection markers, detection substrates and the like. The detection reagent kit may be a diagnostic device *in vitro.*

**The main advantages of the invention include:**
(a) The PD-L1 protein antibody of the present invention has a novel and unique epitope, targeting pan IgC segment. It is different from known PD-L1 monoclonal antibodies that all bind to IgV domain, such as atezolizumab, durvalumab, BMS-963559, and avelumab.
(b) The PD-L1 protein antibody of the present invention can specifically recognizes membrane-bound type PD-L1 and avoids binding to sPD-L1 to the most extent.
(c) The PD-L1 protein antibody of the present invention is against tumors that secrete sPD-L1 and have resistance to PD-L1-blocking-antibody accordingly.
(d) The PD-L1 protein antibody of the present invention has robust ADCC activity.
(e) The PD-L1 protein antibody of the present invention has anti-tumor activity.
(f) The PD-L1 antibody of the present invention may be used in combination with other anti-tumor agents, or provides an alternative approach to tumor resistance to PD-L1 blocking antibody, in order to further improve efficacy in cancer immunotherapy.

The present invention will be further illustrated below with reference to the specific examples. It is to be understood that these examples are for illustrative purposes only and are not intended to limit the scope of the invention. For the experimental methods in the following examples, in which the specific conditions are not specifically indicated, they are performed under routine conditions, e.g., those described by Sambrook. et al., in Molecule Clone: A Laboratory Manual, New York: Cold Spring Harbor Laboratory Press, 1989, or as instructed by the manufacturers, unless otherwise specified. Unless indicated otherwise, parts and percentage are weight parts and weight percentage.

### Primer sequences and expression host cells

**Table 1. human or murine PD-L1/PD-1 specific primers**

| **Expression host cells** | **Name** | **Sequence(5'-3')** | **SEQ ID No:** |
|---|---|---|---|
| CHO-K1 | hPD-L1-F1 | GCGTCGGCTAGCGCCACCATGAGGATATTTGCTGTCT | 1 |
| MC38 | hPD-L1-R1 | GCGCCACTCGAGTTACGTCTCCTCCAAATGTGT | 2 |
| CHO-K1 | pan IgC-F2 | GCCCCATACAACAAAATCAAC | 3 |
| MC38 | pan IgC-R2 | GTTGATTTTGTTGTATGGGGCTGCGTTCAGCAAATGCCAG | 4 |
| MC38 | hPD-L1-3-R3 | GCGTCGCTCGAGTTAATCTCCACTCAGGACTTG | 5 |
| | hPD-L1-9-R3 | GCGTCGCTCGAGTTATTCTCCCAAGTGAGTCCTTTC | 6 |
| CHO-K1 | hPD-1-F4 | GCGTCGGCTAGCGCCACCATGCAGATCCCACAGGCGC | 7 |
| MC38 | hPD-1-R4 | GCGCCACTCGAGTCAGAGGGGCCAAGAGCAGTG | 8 |
| CHO-K1 | hPD-L2-FS | GCGTCGGCTAGCGCCACCATGATCTTCCTCCTGCTAATG | 9 |
| MC38 | hPD-L2-RS | GCGCCACTCGAGTCAGATAGCACTGTTCACTTC | 10 |

| | | | |
|---|---|---|---|
| Note: h stands for human. | | | |

### Host cells for gene expression and experimental use

**Table 2. Host cells for gene expression and experimental use**

| **Cell lines for gene expression** | **Applications of reagents** |
|---|---|
| CHO-K1 and MC38 cells expressing hPD-L1 (membrane type) | PD-L1 antibody detection; Cell interaction assay |
| CHO-K1 and MC38 cells expressing hPD-1 (membrane type) | Cell interaction assay |
| CHO-K1 cells expressing hPD-L1 pan-IgC segment | Immunogen (mouse immunization); PD-L1 antibody detection |
| MC38 cells expressing hPD-L1 pan-IgC segment | PD-L1 antibody detection |
| CHO-K1 and MC38 cells expressing hPD-L2 (membrane type) | PD-L1 antibody detection (negative control) |
| MC38 cells expressing sPD-L1-3 (secrete form or soluble) | sPD-L1-3 secreting MC38 cell tumor mouse model |
| MC38 cells expressing sPD-L1-9 (secrete form or soluble) | sPD-L1-9 secreting MC38 cell tumor mouse model |

| | |
|---|---|
| Note: h stands for human.; s stands for secretory type or solubility. | |

**Table 3. Antibodies involved in the invention**

| **Antibodies** | **Specificity** |
|---|---|
| atezolizumab | Anti PD-L1(binding to IgV) |
| Mouse monoclonal antibody 130021 | Anti PD-L1(binding to pan IgC) |
| Mouse monoclonal antibody 29E.2A3 | Anti PD-L1(binding to IgV) |
| Mouse monoclonal antibody 24F.10C12 | Anti PD-L2 |
| Mouse monoclonal antibody 2D10 | Anti CD80 |
| Mouse monoclonal antibody Ab #18 | Negative control |
| Mouse monoclonal antibody Ab #4 | Anti PD-L1(binding to pan IgC) |
| Mouse monoclonal antibody Ab #11 | Anti PD-L1(binding to pan IgC) |
| Mouse monoclonal antibody Ab #15 | Anti PD-L1(binding to pan IgC) |
| Mouse monoclonal antibody Ab #19 | Anti PD-L1(binding to pan IgC) |
| Mouse monoclonal antibody Ab #23 | Anti PD-L1(binding to pan IgC) |
| mIgG | Negative control |

| | |
|---|---|
| Note: m stands for murine. | |

**Cell lines and cell culture conditions** MC38 is a mouse colon cancer cell, and HEK293 is a human embryonic kidney cell, which were both obtained from Gaining biological, China, and cultured in DMEM plus 10% FBS. CHO-K1, a Chinese hamster ovary cells were from Hua biological, China, and cultured in RPMI1640 + 10%FBS medium .

### Cell-fluorescent antibody labeling and flow cytometry analysis

MC38/CHO-K1 cells expressing either PD-L1 or pan IgC were used as positive detection cells. MC38/CHO-K1 wild type cells and PD-L2-expressed MC38/CHO-K1 cells were used as negative control. About 1 × 10⁵ negative and positive cells were stained with either sera from immunized mice (1:100, 1:1000, 1:10000 dilutions) or culture supernatant (50 µl) from hybridoma, or mouse monoclonal antibody (concentrations as indicated in the figures), or other humanized antibodies (concentrations as indicated in the figures), and incubated at 4°C for 15 min. After washed with PBS, the cells were stained with a PE conjugated goat anti mouse IgG (BD biosciences) and incubated in dark place at 4°C for 15 min. After washed with PBS, the stained cells were analyzed by a flow cytometry (NovoCyte, Acebio, Hangzhou).

### PD-L1 cell ELISA, PD-L1 protein direct ELISA, and secretory type sPD-L1 indirect ELISA

Cell ELISA was established to screen PD-L1 monoclonal antibody. On 96-well cell culture plates, about 5000/well positive cells per wells were plated and incubated in a 37 °C CO₂ cell incubator for 2 days. After the cells were washed with PBS twice, and the culture plate was centrifuged at 1500 rpm for three minutes. Cells were washed for two times.Sera of immunized mice or culture supernatant of monoclonal antibody hybridoma were added and the plate was incubated at 4°C for one hour. Plates were washed with PBS twice and incubated with a goat anti-mouse IgG HRP (Proteintech, Wuhan; 1:1000 dilutions) at 4°C for thirty minutes. After washed with PBS twice, plates were developed with TMB . Chromogenic reaction was stopped with 1M HCl. Plates were read at an optical density (O.D.) of 450 nm by a plate reader (Synergy LX, Biotek, USA), and reading values were used to detect whether or not sPD-L1 were present in supernatants.

Direct ELISA was established to assess whether or not PD-L1 antibody binds to sPD-L1. As conducted before, 50 ng of either recombinant PD-L1-3-HIS or PD-L1-9-HIS were coated in 96 well plates with high protein binding affinity overnight at 4°C. The supernatants were discarded and plates were washed with 100 µl/well PBS + Tween 20 (0.05 %) three times and blocked with 100 µl/well PBS + 1 % BSA for four hours at room temperature. After the supernatants were discarded, plates were incubated with 50 µl/well culture supernatant of monoclonal antibody hybridoma overnight at 4°C. After the supernatants were discarded, plates were washed with 100 µl/well PBS + Tween 20 (0.05 %) five times, and further incubated with a goat anti mouse IgG-HRP (Proteintech; 1:1000 dilutions) for two hours at room temperature. Plates were developed with TMB. After adding TMB for color development, the absorption value of 450 wavelength is used to detect whether the antibody binds to sPD-L1. Secretory type sPD-L1 (native form) ELISA was used to clarify affinity of PD-L1 antibody to sPD-L1. As conducted before, native forms of sPD-L1 were expressed and secreted by MC38 cells. Cell culture supernatants containing sPD-L1 were used to examine PD-L1 antibody affinity. Purified PD-L1 antibodies(0.2 µg/well) were coated overnight at 4°C. 100 µl/well culture supernatants of MC38 cells expressing sPD-L1-3 and sPD-L1-9 were added. Plates were incubated overnight at 4°C and washed. 0.1 µg/100 µl/well biotinylated PD-L1 antibody (Biolegend, USA) was added and incubated at room temperature for two hours. Plates were washed and incubated with 100 µl/well streptavidin-HRP (Jackson ImmunoResearch, USA; 1:20,000 dilutions) at room temperature for two hours. Plates were developed with 70 µl/well TMB and stopped with 35 µl/well 1M HCl. Binding affinity of antibody to sPD-L1 was analyzed by absorbance at 450 nm.

### Cell interaction assay

In order to analyze impact of PD-L1 antibody on interaction of PD-L1 with PD-1, MC38 and CHO-K1 cells were transfected to express human PD-L1 and PD-1, respectively. Following the instructions from EMD (USA) on cell fluorescence staining, PD-L1 expressed MC38 and CHO-K1 cells were labeled with PKH67, and PD-1 expressed MC38 and CHO-K1 cells were labeled with PKH26. Two different labeled cells expressing either PD-L1 or PD-1 were co-cultured in absence or presence of PD-L1 antibody or antibody isotype, respectively, for one hour. Impact of PD-L1 antibody on interactions of PD-L1 with PD-1 was examined by flow cytometry. Double positive cell population indicates binding of PD-L1 to PD-1. In addition, PD-L1 expressed MC38 and CHO-K1 cells were labeled with PKH26, PD-1 expressed MC38 and CHO-K1 cells were labeled with PKH67, the different labeled MC38 or CHO-K1 cells with expression of PD-L1 were co-cultured, and the different labeled MC38 or CHO-K1 cells with expression of PD-1 were also co-cultured. The double positive cell population indicates non-specific binding (background control).

### ADCC assay

Jurkat NFAT-Luciferase-CD16 were provided by ICAREAB (Suzhou) and used to detect antibody Fc induced CD16 activation and intracellular activation of NF-AT signal pathway and luciferase production, and further analyzed antibody Fc effector function in quantity. In presence of human PD-L1 antibody at different concentrations, about 1.25 × 10⁴/well PD-L1-expressed CHO-K1 target cells were mixed with the Jurkat effector cells expressing human FcyRIIIa at a ratio of 1:10. The cell mixture was incubated for 10 hours. 75 µl /well Bright Lite^{™} (Vazyme, Nanjing) luciferase substrate were added and incubated at room temperature for 5-30 minutes. Antibody Fc induced ADCC activity was detected in quantity by a luminometer (Synergy LX).

### PD-L1 antibody preparation

Antibodies with high affinity to pan IgC segment of PD-L1 were obtained by preliminary production, purification, and identification.

**Table 4. Amino acid sequences of heavy-chain variable region, HCDR, light-chain variable region, and LCDR in anti-PD-L1 antibody of the present invention**

| **Name** | **#19 mouse monoclonal antibody (IgG2bx) and its humanized antibody sequences** | **SEQ ID No:** | **Position** |
|---|---|---|---|
| VH of mouse monoclonal antibody #19 | EVQLQQSGPELVKPGASVKISCKASGSAFSSSWMNWLKQRPGQGLEWIGR IYPGDGDTNYNGKFKGKATLTADKSSSTAYMQLSSLTSVDSAVYFCARRRA NHYGSNWYFDVWGAGTTVTVSS | 11 | |
| VH-CDR1 | GSAFSSS | 12 | aa26-32 |
| VH-CDR2 | IYPGDGDT | 13 | aa51-58 |
| VH-CDR3 | ARRRANHYGSNWYFDV | 14 | aa97-112 |
| VL of mouse monoclonal antibody #19 | NIVLTQSPASLAVSLGQRATISCRASESVDSYGKSFMHWYQQKPGQPPKLLI YLASNLESGVPARFSGSGSRTDFTLTIDPVEADDAATYYCQQNNEDPYTFG GGTKLEIK | 15 | |
| VL-CDR1 | ESVDSYGKSF | 16 | aa27-36 |
| VL-CDR2 | LAS | 17 | aa54-56 |
| VL-CDR3 | OONNEDPYT | 18 | aa93-101 |
| VH1 | EVQLVQSGAEVKKPGSSVKVSCKASGSAFSSSWMNWLRQAPGQGLEWM GRIYPGDGDTNYNQKFQGRVTLTADKSTSTAYMELSSLRSVDTAVYFCARR RANHYGSNWYFDVWGQGTTVTVSS | 20 | |
| VH2 | QVQLVQSGAEVKKPGASVKVSCKASGSAFSSSWMNWLRQAPGQGLEWIG RIYPGDGDTNYNQKFQGRVTLTADSSISTAYMELSRLRSVDTVVYFCARRR ANHYGSNWYFDVWGQGTTVTVSS | 21 | |
| VH3 | EVQLVQSGAEVKKPGASVKVSCKASGSAFSSSWMNWLRQAPGQGLEWM GRIYPGDGDTNYNQKFQGRVTLTADKSISTAYMELSRLRSVDTAVYFCARR RANHYGSNWYFDVWGQGTTVTVSS | 22 | |
| VH4 | EVQLVQSGAEVKKPGSSVKVSCKASGSAFSSSWMNWLRQRPGQGLEWIG RIYPGDGDTNYNGKFKGRVTLTADKSSSTAYMELSSLRSVDTAVYFCARRR ANHYGSNWYFDVWGAGTTVTVSS | 23 | |
| VH5 | EVQLVQSGAEVKKPGASVKVSCKASGSAFSSSWMNWLRQRPGQGLEWIG RIYPGDGDTNYNGKFKGRVTLTADKSISTAYMELSRLRSVDTAVYFCARRR ANHYGSNWYFDVWGAGTTVTVSS | 24 | |
| VH6 | EVQLQQSGAEVKKPGSSVKVSCKASGSAFSSSWMNWLKQRPGQGLEWIG RIYPGDGDTNYNGKFKGRVTLTADKSSSTAYMQLSSLRSEDTAVYFCARRR ANHYGSNWYFDVWGAGTTVTVSS | 25 | |
| VL1 | NIVLTQSPATLSLSPGERATLSCRASESVDSYGKSFMHWYQQKPGQPPKLLI YLASNLETGIPARFSGSGSRTDFTLTISSLEPEDFAVYYCQQNNEDPYTFGGG TKLEIK | 26 | |
| VL2 | NIVLTQSPSSLSASVGDRVTITCRASESVDSYGKSFMHWYQQKPGQPPKLLI YLASNLESGVPSRFSGSGSRTDFTLTISSLQPEDFATYYCQQNNEDPYTFGG GTKLEIK | 27 | |
| VL3 | NIVLTQSPATLSLSPGERATLSCRASESVDSYGKSFMHWYQQKPGQPPKLLI YLASNLETGIPARFSGSGSRTDFTLTIDSLEPEDFAVYYCQQNNEDPYTFGGG TKLEIK | 28 | |
| VL4 | NIVLTQSPASLSASVGDRVTISCRASESVDSYGKSFMHWYQQKPGQPPKLLI YLASNLESGVPARFSGSGSRTDFTLTISSLQPEDFATYYCQQNNEDPYTFGG GTKLEIK | 29 | |
| VL5 | NIVLTQSPASLSASVGDRVTISCRASESVDSYGKSFMHWYQQKPGQPPKLLI YLASNLESGVPARFSGSGSRTDFTLTIDSLQPEDFATYYCQQNNEDPYTFGG GTKLEIK | 30 | |

| | | | |
|---|---|---|---|
| Note: VH: variable region of heavy chain; VH-CDR: Complementarity Determining Regions of heavy chain; VL: variable region of light chain; VL-CDR: Complementarity Determining Regions of light chain variable. As used herein, H6L2 represents the humanized PD-L1 antibody comprising heavy-chain variable region as VH6 and light-chain variable region as VL2. | | | |

### Example 1: PD-L1 antibody preparation

### Step 1. Generation of cell lines expressing either PD-L1 or PD-1, or PD-L1-pan-IgC

Human or murine PD-L1, PD-1, and PD-L2 DNA templates were obtained from You biological, China. Various genes and gene fragments were amplified by PCR using the gene specific primers (as shown in Table 1).

PCR products were inserted into pcDNA4 vector (Thermofisher, USA) using restrict enzymes and T4 DNA ligase. The pcDNA4 vector expressing genes and gene fragments were transfected into different host cells, and gene-expressed cell lines were generated by resistant selection. Wherein, expressed vector comprising gene fragments of PD-L1, pan IgC, PD-L2, or PD-1 was transfected into CHO-K1 and MC38 cells; vector expressing fragment comprising either sPD-L1-3 or sPD-L1-9 was transfected into MC38 cells; vector expressing fragment comprising gene fragments of either sPD-L1-3-HIS or sPD-L1-9-HIS was transfected into HEK293 cells. Host cells that express either PD-L1 or PD-L1-pan-IgC, or PD-L2, or PD-1 and their applications are listed in Table 2.

### Step 2. Monoclonal antibody hybridoma preparation

Two of 5 mice/group were immunized using either a hPD-L1 (aa19-290) expressed vector or pan-IgC highly expressed CHO-K1 cells. Sera of immunized mice were diluted at 1:100, 1:1000, and 1:10000. The antibody titers and specificities were detected using hPD-L1 expressed MC38 cells (positive control cells) or hPD-L2 expressed MC38 cells and MC38 parental line (negative control cells) by flow cytometry. Cell fusions were conducted from positive responders, and binding affinities and specificities of antibodies in culture supernatants were examined using positive and negative cell controls by ELISA and flow cytometry. After primary and confirmatory screenings, qualified clones were chosen for next round sub-cloning selection, monoclonal antibody hybridoma were further generated and obtained. Humanized PD-L1 mouse monoclonal antibodies with non or low affinity to sPD-L1 were chosen by binding detection of sPD-L1 protein, in particular, those selected were mouse IgG2a and IgG2b subtypes with ADCC and CDC function.

### Step 3. Anti-PD-L1 monoclonal antibody preparation

Monoclonal antibody hybridoma were i.p. injected into mice, and ascites were collected. The antibodies were obtained by purification of protein A affinity chromatography.

### Step 4. DNA sequencing of heavy-chain and light-chain variable regions of anti human PD-L1 antibody, and its humanization, expression, and purification

Briefly, total RNA were isolated from mouse monoclonal antibody hybridoma. cDNA of variable regions of heavy chain and corresponding light chain were generated using gene-specific primers by PCR, and further inserted into pCR^{™} vector by TOPO TA cloning and sequenced. Variable regions of mouse monoclonal antibody were humanized by CDR grafting and back mutation, and grafted into human IgG1, κ. After humanized antibody sequences were synthesized, pcDNA3.1 vector (Thermofisher) expressing either heavy chain or light chain of antibody was constructed; meanwhile, an expression vector of a chimeric antibody was constructed as a control. Vectors for expression of heavy chain and light chain were transfected into HEK293 cells. Cells secreting antibody were cultured and amplified. Antibody in culture supernatant were isolated by protein A affinity chromatography.

### Example 2: PD-L1 mouse monoclonal antibody selection

Monoclonal antibodies with high affinity to hPD-L1 and pan-IgC were selected by flow cytometry. MC38 cells expressing either hPD-L1 or pan-IgC were used as positive detection cells, whereas, both hPD-L2 expressed and parental MC38 cells were used as negative detection cells.

50 µl/well of hybridoma culture supernatant or 100 ng/well mouse monoclonal antibody were mixed with 1 × 10⁵ of either positive or negative detection cells at 4 °C for 15 min. After washed with PBS, the cells were stained with a PE conjugated goat anti mouse antibody (BD biosciences) at 4 °C for 15 min in dark place. The cells are washed with PBS and further examined by flow cytometry.

As shown in Figure 3A, PD-L1 mouse monoclonal antibody #130021 (R&D systems) bound to both PD-L1 and pan-IgC. PD-L1 mouse monoclonal antibody #29E.2A3 (Biolegend) only bound to PD-L1 but not pan-IgC, indicating that its recognition region is located in IgV As shown in Figure 3B, 5 of 33 monoclonal antibodies, which bound to both PD-L1 and pan-IgC, were selected by flow cytometry. They were mAb#4, mAb#11, mAb#15, mAb#19, and mAb#23, respectively.

### Example 3: Affinity of PD-L1 mouse monoclonal antibody to PD-L1 recombinant protein, and impact of sPD-L1 on the its affinity

Binding affinities of PD-L1 antibodies to recombinant PD-L1 were examined by PD-L1 protein direct ELISA. PD-L1-3-HIS and PD-L1-9-HIS recombinant proteins were expressed in HEK293 cells, and the sPD-L1-HIS recombinant proteins were purified using Ni column from the supernants. 50 ng/well of either rPD-L1-3-HIS or rPD-L1-9-HIS were coated onto 96 well plates with high protein binding affinity at 4 °C overnight. After supernatants were discarded, plates were washed with 100 µl/well PBS + Tween 20 (0.05 %) three times and further blocked with 100 µl/well PBS + 1 % BSA at room temperature for four hours. After supernatants were discarded, 100 ng/100 µl/well mouse monoclonal antibodies were added and incubated at 4 °C overnight. After supernatants were discarded, plates were washed with 100 µl/well PBS + Tween 20 (0.05 %) five times and further incubated with a goat anti mouse IgG-HRP (Proteintech, 1:1000 dilutions) two hours at room temperature. Plates were developed with TMB and read at an optical density of 450 nm for detecting whether or not antibodies bind to sPD-L1. Binding affinities of mAb#4, mAb#11, mAb#15, mAb#19 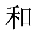 mAb#23 to the recombinant PD-L1 (rPD-L1) were examined by direct ELISA.

As shown in Figure 4A, all of the five mouse monoclonal antibodies did not bind to rPD-L1-3-HIS; binding affinity of mouse monoclonal antibody #19 was the lowest for rPD-L1-9-HIS among those antibodies.

Blocking effects of rPD-L1-3-HIS and rPD-L1-9-HIS on the five PD-L1 monoclonal antibodies were examined by flow cytometry. 200 ng of either rPD-L1-3-HIS (14 fold molar excess of the antibodies) or rPD-L1-9-HIS (10 fold molar excess of the antibodies) were pre-mixed and pre-incubated with 100 ng of mAb#4, mAb#11, mAb#15, mAb#19, and mAb#23, respectively, at 4 °C for two hours. The mixtures were further incubated with PD-L1 expressed MC38 cells at 4 °C for 30 min.

As shown in Figure 4B, rPD-L1-3-HIS can not block the binding of the five antibodies to PD-L1 expressed on the cells, rPD-L1-9-HIS didn't block binding of mAb #4, mAb #15, and mAb #23 but partially inhibited binding of mAb #11 and #19 to PD-L1 expressed on the cells. It indicates that the mouse monoclonal antibodies are able to circumvent secretory type PD-Ll-3-HIS completely and PD-Ll-9-HIS partially.

Mouse monoclonal antibody #19 was selected from the five screening-out antibodies, and impact of rPD-L1-9-HIS on its binding affinity to PD-L1 was further examined. 100 ng of the antibody #19 were pre-incubated with rPD-L1-9-HIS at different concentrations as indicated at 4 °C for two hours and further incubated with PD-L1 expressed MC38 cells at 4 °C for 30 min. Impact of rPD-L1-9-HIS on binding function of the PD-L1 mouse monoclonal antibody #19 to PD-L1 was analyzed by flow cytometry.

As shown in Figure 4C, mouse monoclonal antibody #19 was able to bind to PD-L1 in presence of rPD-L1-9-HIS at 40 fold molar excess of the antibody. It further indicates that the mouse monoclonal antibody can partially circumvent rPD-L1-9-HIS.

Binding affinity of PD-L1 mouse monoclonal antibody #19 to native forms of sPD-L1 was examined by sPD-L1 indirect ELISA. MC38 cells were transfected to express and secrete native forms of sPD-L1. Cell culture supernatants contain sPD-L1. 0.2 µg/well of purified PD-L1 monoclonal antibody was coated overnight at 4°C. 100 µl/well MC38 cell culture supernatant including either sPD-L1-3 or sPD-L1-9 was added and incubated overnight at 4°C. Plates were washed and incubated with 0.1 µg/100 µl/well biotinylated PD-L1 antibody (Biolegend) at room temperature for two hours. Plates were washed and incubated with 100 µl/well streptavidin-HRP (1:20,000 dilutions, Jackson ImmunoResearch) at room temperature for two hours. Chromogenic reaction was developed with 70 µl/well TMB substrate and subsequently stopped with 35 µl/well 1M HCl. Affinity of antibody to sPD-L1 was examined by absorbance at 450 nm.

Binding affinities of monoclonal antibody #19 to either sPD-L1-3 plus sPD-L1-9 (A) or sPD-L1-3 (B), or sPD-L1-9 (C) in culture supernatant were examined by ELISA. Anti-PD-L1^{total} recognized both sPD-L1-3 and sPD-L1-9, whereas, clone #130021 only bound to sPD-L1-9.

As shown in Figure 5, results indicate that PD-L1 mouse monoclonal antibody #19 specifically recognized membrane-bound PD-L1, but didn't binds to native form of sPD-L1-3, and its affinity to native form of sPD-L1-9 was much lower than clone #130021 (Notably, in Figure 3A and 3B, flow cytometry assays showed that affinity of the antibody to membrane-bound PD-L1 was much weaker than PD-L1 mouse monoclonal antibody #19), e.g., circumventing secretory type sPD-L1 (sPD-L1-3 and sPD-L1-9) to the most extent.

### Example 4: MC38 tumor mouse model for examining antibody activity

MC38 cells that express either membrane-bound PD-L1 or sPD-L1 were mixed at ratio of 20: 1. About 5 × 10⁵ cells/mouse were s.c. injected into C57BL/6J, and there were six mice per group. At day 7 post-injection, tumors were formed, 100 µg/mouse of either a mouse antibody isotype or mouse monoclonal antibody #19 (PD-L1 antibody treatment) were i.p. injected at day 7, 10, and 15, three times in total. Tumor size and mouse survival were regularly examined. Tumor volume = (tumor length × width²)/2. Whether or not there are any significant differences between two groups in tumor size and mouse survival were analyzed by Student's T test and Log-rank test.

As shown in Figure 6, results indicate that the PD-L1 specific antibody treatment has effects of anti-resistance and anti-tumor in mouse tumor model of MC38 cells expressing PD-L1 and sPD-L1.

### Example 5: Binding activity of humanized mouse monoclonal antibody #19

Mouse monoclonal antibody #19 was humanized and obtained. Amino acid sequences of mouse monoclonal antibody #19 and different heavy-chain (A) and light-chain (B) Fab of its humanized antibodies are shown in Figure 7 and Table 4. Wherein, VH1, VH2, VH3, VH4, VH5, and VH6 represent heavy-chain variable regions of humanized mouse monoclonal antibody #19; VL1, VL2, VL3, VL4, and VL5 represent light-chain variable regions of humanized mouse monoclonal antibody #19.

### 5.1. Affinity of humanized mouse monoclonal antibody #19 to membrane-bound type and secretory type PD-L1, and pan IgC

Affinities of different combinations of heavy (H) and light (L) chains to membrane and secretory types of PD-L1, as well as pan IgC were examined, and high affinities of the combinations of the heavy (H) and light (L) chains were chosen. Wherein, affinities of H6L2, H6L5, H4L4, H5L4, H6L4, H5L5, and H5L4 were preferential.

Wherein, affinities of the different combinations of the heavy (H) and light (L) chains (H6L2, H6L5, H4L4, H5L4) to membrane-bound type and secretory type PD-L1, as well as pan IgC were examined as shown in Figure 8.

Affinities of humanized mouse monoclonal antibodies to membrane-bound PD-L1 (A) and pan-IgC (B) were examined by flow cytometry.

MC38 cells were transfected to secrete native forms of sPD-L1-3 and sPD-L1-9 in culture supernatants, binding of humanized mouse monoclonal antibodies to secretory types of sPD-L1 (8C and 8D) were examined by ELISA, compared to mouse monoclonal antibody #19 and its chimeric antibody, antibody 130021, and anti-PD-L1^{total}.

Results showed that humanized combinations of heavy and light chains of mouse monoclonal antibody #19 (H6L2, H6L5, H4L4, and H4L5) bind to PD-L1 and pan IgC, and they didn't bind to sPD-L1-3, and their affinities to sPD-L1-9 were lower.**5**.**2**. **Affinity of**

### humanized mouse monoclonal antibody #19 to PD-L2 (human)

Affinities of the different combinations of heavy (H) and light (L) of humanized monoclonal antibody #19 to membrane-bound PD-L2 were examined by flow cytometry.

As shown in Figure 9, H6L2 and H6L5 didn't bind to PD-L2, and H5L4 had weaker binding to PD-L2 and CHO-K1 parental cells.

### 5.3. Affinity of humanized mouse monoclonal antibody #19 to CD80 (human)

Affinities of the different combinations of heavy (H) and light (L) of humanized monoclonal antibody #19 to membrane-bound CD80 were examined by flow cytometry.

As shown in Figure 10, H6L2 and H6L5 didn't bind to CD80.

### 5.4. Affinity of humanized mouse monoclonal antibody #19 H6L2 to membrane-bound type PD-L1

### 5.4.1 Affinity of humanized antibody H6L2 to membrane-bound type PD-L1 in presence or absence of rPD-L1-9-HIS

Affinity of humanized antibody H6L2 to membrane-bound PD-L1 was examined by flow cytometry, in comparison with avelumab and atezolizumab; Impact of rPD-L1-9-HIS at different concentrations on affinity of H6L2 was also examined, in comparison with avelumab and atezolizumab.

As shown in A of Figure 11, EC50 of H6L2, avelumab, and atezolumab were 0.62, 0.17, and 0.21 µg/ml, respectively.

As shown in B and C of Figure 11, affinity of humanized antibody H6L2 was higher than avelumab and atezolizumab in presence of sPD-L1-9-HIS.

### 5.4.2 Affinity of humanized antibody H6L2 to membrane-bound type PD-L1 in presence or absence of rPD-L1-3-HIS

Affinity of humanized antibody H6L2 to membrane-bound PD-L1 was examined by flow cytometry, in comparison with avelumab and atezolizumab, impact of different concentrations of rPD-L1-3-HIS on affinity of H6L2.

As shown in Figure 12, the experimental results showed that the affinity of humanized antibody H6L2 against sPD-L1-3 was higher than avelumab and atezolizumab.

### 5.5 Blocking effect of humanized antibody H6L2 on binding of PD-1 to PD-L1

To analyze the interaction of PD-L1 with PD-1, CHO-K1 cells were transfected and expressed human PD-L1 and PD-1, respectively. PD-L1 expressed CHO-K1 cells were labeled with PKH67, whereas, PD-1 expressed CHO-K1 cells were labeled with PKH26. The two different labeled cells were co-cultured in presence or absence of H6L2 for one hour. Different concentrations of antibody are indicated in figure. Impact of the antibody on intercellular interaction of PD-L1 with PD-1 was analyzed by flow cytometry. Double positive cell population indicates binding of PD-L1 to PD-1. In the meanwhile, PD-L1 expressed CHO-K1 cells were labeled with PKH26, PD-1 expressed CHO-K1 cells were labeled with PKH67, the different labeled CHO-K1 cells with expression of PD-L1 were co-cultured, and the different labeled CHO-K1 cells with expression of PD-1 were also co-cultured. The double positive cell population indicates non-specific binding (background control). Results are shown in Figure 13

Results showedthat H6L2 can not block intercellular binding of PD-L1 to PD-1.

### 5.6 ADCC of humanized antibody H6L2, and impact of secretory type sPD-L1 on its ADCC

As shown in 14, in Jurkat-CD16-NFAT-Luciferase system, the ability of humanized antibody #19 induced Luciferase activity in absence (A) or presence (B) of sPD-L1 secretory types (recombinant protein PD-L1-3-HIS or sPD-L1-9-HIS) were examined, in comparison with avelumab.

Results showed that humanized antibody H6L2 induced ADCC activity and its ability against sPD-L1 were superior to Avelumab.

### Example 6: Anti-tumor effect of humanized mouse monoclonal antibody #19 H6L2

3 to 5 × 10⁵ cells/mouse MC38 cells that express PD-L1 were s.c. injected into C57BL/6J, and there were five mice/group. At day 7 post-injection, tumors were formed, 100 µg/mouse of either humanized antibody H6L2 or avelumab were i.p. injected, or 100µl/mouse PBS i.p. injected as control group at day 7, 10, and 13, three times in total.

As shown in Figure 15, A. Tumor size, average tumor volume of each group ± standard error. B. Survival rate. Whether or not there were any significant differences among groups in tumor sizes and mouse survival were analyzed by Student's T test and Log-rank test.

Results showed that humanized antibody H6L2 had a statistically significant antitumor effect, and had significantly anti-tumor effect in comparison with avelumab.

### Comparison example 1. Binding of human PD-L1 antibody atezolimumab to native forms of sPD-L1 by indirect ELISA

Binding of atezolizumab to native forms of sPD-L1 was examined by secretory type sPD-L1 indirect ELISA. MC38 cells were transfected to express sPD-L1-3 and sPD-L1-9, respectively. sPD-L1 in cell culture supernatants that was pre-incubated with atezolizumab were analyzed for binding ability of the antibody by ELISA. Cell culture supernatants containing sPD-L1-3 and sPD-L1-9 was pre-mixed and incubated with 1.5 µg/ml of either atezolizumab or human IgG1 overnight (ate treated or hIgG1 treated as shown in figure), and the atezolizumab antibodies were depleted with protein G argarose (Thermofisher) by centrifugation. Supernatant that contained either sPD-L1-3 or sPD-L1-9 and had no any pre-incubation was used as positive control (sPD-L1-3 or sPD-L1-9 as indicated in figure). BL indicates supernatants without any sPD-L1 and stands for background.

As shown in Figure 16, results indicated that atezolizumab was able to binds to both sPD-L1-3 and sPD-L1-9.

### Comparison example 2. Blocking effects of human PD-L1 antibody avelumab and atezolizumab on binding of PD-1 to PD-L1, and impact of sPD-L1 on the blocking effects

In order to examine blocking effects of human PD-L1 antibody avelumab and atezoluzumab on binding of PD-1 to PD-L1, and impact of sPD-L1 on the blockade. CHO-K1 cells were transfected to express either PD-L1 or PD-1, respectively. PD-L1 expressed CHO-K1 cells were labeled with PKH67, whereas, PD-1 expressed CHO-K1 cells were labeled with PKH26. The two different labeled cells were co-cultured in absence or presence of either avelumab or atezolizumab for one hour. Different concentration of the antibodies were shown in Figure 17. Impacts of the two antibodies on intercellular interactions of PD-L1 with PD-1 were examined by flow cytometry. Double positive cell population indicated binding of PD-L1 to PD-1.

Wherein, A and B, impacts of avelumab and atezolizumab on intercellular interactions of PD-1 with PD-L1. C and D, Impacts of sPD-L1 on blockade of avelumab and atezolizumab. Antibody concentration is 100 ng/datum point, rPD-L1-9-HIS concentration are shown in Figure. In the meanwhile, PD-L1 expressed CHO-K1 cells were labeled with PKH26, whereas, PD-1 expressed CHO-K1 cells were labeled with PKH67. The two different labeled CHO-K1 cells expressing PD-L1 were co-cultured, the two different labeled CHO-K1 cells expressing PD-1 were co-cultured, double positive cell population indicated non-specific binding (background control).

Results showed that more than 1 µg/ml of avelumab and atezolizumab achieved complete blockade on intercellular binding of PD-L1 to PD-1, sPD-L1 was able to reduce or abolish the blocking effect of avelumab and atezolizumab, thus the complete blockade couldn't be achieved.

The results from comparison experiments indicated that high concentrations (> 1µg/ml) of avelumab and atezolizumab was able to completely block interaction of PD-L1 with PD-1, and due to their binding to native forms of sPD-L1, they showed reduced blocking effects, leading to resistance to the blockade.

### Discussion

Antibody Fc region binds to its receptor FcyRs, which are expressed in a serises of different immune cells, such as NK cells, monocytes, neutraphils, and macrophages. Interactions of Fc with FcγRs play irreplaceable roles in ADCC, CDC, antibody metabolism and titer. ADCC activity can be significantly increased by engineering Fc CH2 region via DEL replacement.

In regarding to the role of PD-L1 blocking antibody Fc in anti-tumor effect, a study showed that a PD-L1 blocking antibody (clone 14D8) was replaced with different murine Fc with distinct functions, there were no differences in their specificities, affinities, and metabolism, however, their anti-tumor effects were significantly different. 14D8 with an activating IgG2a Fc (e.g., 14D8/IgG2a) specifically bound to and activated FcyRs and inhibited tumor growth, whereas, 14D8/IgG1 bound to inhibitory FcyRs and 14D8/IgG1-D265A lost binding ability to FcyRs and these two types of antibodies were significantly deprived of anti-tumor effect. In addition, anti-tumor effect of another blocking PD-L1 antibody (clone 10F.9G2) was significantly reduced in mouse tumor mode in which activating FcyRs were absent but inhibitory Fc γ RIIb was retained, compared with wild-type mice . The antibody was widely applied to anti-tumor mechanism study. These studies strongly suggests that ADCC/CDC induced by Fc of PD-L1 blocking antibody play critical roles in anti-tumor effect. On the other hand, it suggests limited anti-tumor effect from blockade of PD-L1 blocking antibody on the interaction of PD-L1 with PD-1. Our studies also showed that complete blockade could be achieved only under high concentration of PD-L1 blocking antibody.

NK cells are innate immune effector cells, and they are able to lyse MHC class I negative tumor cells and virus infected cells and orchestrate innate immunity. NK cells constitutively express lytic machinery. Therefore, they are able to kill target cells without any pre-activation and play important role in immune surveillance against aberrant cells and tumor cells.

ADCC is primarily dependent of killing activity of NK cells. Different from T cells expressing PD-1, NK cells, under multiple conditions and showing up-regulation of activation/regulatory markers, consistently lack PD-1 expression detected by flow cytometry, qRT-PCR, and RNAseq. This feature is helpful for NK cells to circumvent PD-1/PD-L1 pathway and to kill PD-L1 expressed tumor cells. Furthermore, cytotoxic antibodies targeting tumor have been successfully developed, and they are able to induce ADCC and clinically and effectively treat cancer patients, such as rituximab, daratumumab, trastuzumab, and cetuximab. In comparison with avelumab, our antibody has robust induction of ADCC and significant anti-tumor effect. Taken together, it includes utilization of ADCC and NK cells targeting PD-L1-expressed tumor cells for direct killing effect but not limited to blockade of PD-L1 function, recovery of T cell function and indirect killing effect. Therefore, it is possible for potential increase in therapeutic efficacy.

Monoclonal antibodies of #130021, #4, #15, and #23 had lower affinity to membrane-bound type PD-L1 but higher affinity to sPD-L1-9 (full-length of extracellular domain of PD-L1), compared to #19. This suggests that antibody affinity to membrane-bound PD-L1 can not be applied to determine the binding activity to pan-IgC region of sPD-L1 and vice versa. In addition, tertiary structure between membrane-bound and secreted proteins might not be complete same, despite that both proteins share same amino acid sequence. Our experimental results suggested that affinity differences of the antibodies to the two types of the target protein existed, and it might provide opportunity for treating cancer patients that have tumors secreting sPD-L1 and show resistance to PD-L1 blockade.

Based on resistance mechanism related to sPD-L1 and characteristics of amino acid sequence of sPD-L1, and critical roles of PD-L1 blocking antibody Fc in anti-tumor effect, inventor hypothesized and designed a novel epitope that was used as immunogen for inducing and screening PD-L1 specific antibodies. The antibody target pan-IgC of PD-L1 domain and circumvent sPD-L1 to the most extent, and has robust ADCC/CDC activity and anti-tumor effects. This provides an alternative approach to treat cancer patients, particularly for those who have tumors with sPD-L1 secretion and resistance to blockade by PD-L1 blocking antibody, and also for combination treatment with other therapeutic agents, and it is potentially significant for improvement of tumor immunotherapy. All literatures mentioned in the present application are incorporated herein by reference, as though each one is individually incorporated by reference. In addition, it should also be understood that, after reading the above teachings of the present invention, those skilled in the art can make various changes or modifications, and these equivalents also fall in the scope of claims as defined in the appended claims.

## Claims

1. A PD-L1 antibody or an active fragment thereof, wherein the antibody does not block the binding of PD-L1 with PD-1 and specifically binds to pan IgC segment of PD-L1 and has ADCC/CDC functional effects activity.

2. The PD-L1 antibody or an active fragment thereof of claim 1, comprises:
(a) amino acid sequences as shown in SEQ ID NO: 16 of LCDR1, SEQ ID NO: 17 of LCDR2, SEQ ID NO: 18 of LCDR3; and
(b) amino acid sequences as shown in SEQ ID NO: 12 of HCDR1, SEQ ID NO: 13 of HCDR2, SEQ ID NO: 14 of HCDR3;
wherein, the antibody sepecifically binds to pan IgC segment of PD-L1.

3. The PD-L1 antibody or an active fragment thereof of claim 1 or 2, wherein the PD-L1 antibody or an active fragment thereof comprises heavy chain variable region or light chain variable region, the polypeptide sequence of the heavy chain variable region is at least 95% homologous to SEQ ID:11, 20, 21, 22, 23, 24, or 25, the polypeptide sequence of the light chain variable region is at least 95% homologous to SEQ ID:15, 26, 27, 28, 29, or 30.

4. The PD-L1 antibody or an active fragment thereof of any one of claims 1-3, comprises the polypeptides of heavy chain variable regions as shown in SEQ ID:11, 20, 21, 22, 23, 24, or 25, and polypeptides of light chain variable region as shown in SEQ ID:15, 26, 27, 28, 29, or 30.

5. The PD-L1 antibody or an active fragment thereof of any one of claims 1-4, the antibody or its active fragment thereof is chimeric.

6. The PD-L1 antibody or an active fragment thereof of any one of claims 1-5, the antibody or its active fragment thereof is humanized.

7. A recombinant protein, the recombinat protein comprises:
(i) the antibody or its active fragment of any one of claims 1-6; and
(ii) an optional tag sequence to assist expression or purification.

8. A polynucleotide encoding the antibody or its active fragment of any one of claims 1-6, or the recombinant protein of claim 7.

9. A vector, which comprises the polynucleotide of claim 8.

10. A genetically engineered host cell, which comprises the vector of claim 9 or the polynucleotide of claim 8 integrated in the genome.

11. An immunoconjugate, which includes:
(a) an PD-L1 antibody or its active fragment of claim 1; and
(b) a coupling moietyselected from the following group consisting of a detectable label, a drug, a toxin, a cytokine, a radionuclide, or an enzyme, or a combination thereof.

12. A pharmaceutical composition, which comprises:
(a) the PD-L1 antibody or its active fragment of claim 1, or the recombinant protein of claim 2, or the immunoconjugate of claim 11; and
(b) a pharmaceutically acceptable expression vector.

13. A method for detecting PD-L1 protein in samples, comprising the steps of:
(1) samples obtained from subjects;
(2) samples are interacted with the PD-L1 antibody or its active fragment of claim 1;
(3) detemining PD-L1 levels in subjects.

14. An immune cell, the antibody of any one of claims 1-4 is expressed by the immune cell or exposed on extracellular membrane surface of the cell.

15. A method for treating a disease associated with abnormal PD-L1 expression or function, wherein the method comprises administering the pharmaceutically effective amount of the PD-L1 antibody of claim 1, the immunoconjugate of claim 11, or the pharmaceutical composition of claim 12, the immune cells of claim 14, or other combinations thereof, to a subject in need.

16. The method of claim 15, the disease associated with abnormal PD-L1 expression or function is cancers or infection diseases.

17. The method of claim 16, the infection diseases are caused by pathogens selected from the group consisting of HIV, hepatitis virus (a, b, c), and tuberculosis bacillus.
